# EUROPEAN PATENT APPLICATION

(11) **EP 4 545 090 A1**
(43) Date of publication of application: **30.04.2025**
(21) Application number: 23218581.9
(22) Date of filing: 20.12.2023
(51) Int. Cl.: A61K 38/36, A61P 13/12, A61P 29/00, A61P 37/00, A61P 43/00, C07K 16/00

(54) **INTERFERON-A RECEPTOR IMMUNE MODULATOR FOR USE AS A MEDICAMENT**

(30) Priority: 27.10.2023 DE 102023129710; 27.10.2023 DE 102023129694
(71) Applicant: Universität Leipzig, 04109 Leipzig (DE)
(72) Inventor: JAYAKUMAR, Manoharan, 04109 Leipzig (DE); ISERMANN, Berend, 04109 Leipzig (DE)
(74) Representative: Hertin und Partner Rechts- und Patentanwälte PartG mbB

(57) **Abstract**

The invention refers to an interferon-a receptor (IFNAR1) immune modulator for use as a medicament for regulating the interaction of tissue factor (TF) and interferon-a receptor (IFNAR1) in a subject and especially to an IFNAR1 immune modulator for use as a medicament according for treating a tumor disease in a subject by regulating the interaction of TF and IFNAR1 or for treating chronic inflammatory disease, autoimmune diseases and/or interferonopathy in a subject by regulating the interaction of TF and IFNAR1.

## Description

### BACKGROUND OF THE INVENTION

Interferon-a receptor (IFNAR1) is a type I transmembrane protein classified as cytokine receptor. IFNAR1 functions as a ubiquitous membrane receptor which binds endogenous type I interferon (IFN) cytokines. Upon binding, IFNAR1 activates various signaling pathways, including the JAK/STAT, MAPK, PI3K, and Akt signaling pathways.

IFNAR1 has diverse effects. On one hand, in the context of inflammatory diseases, including autoimmunological diseases, IFNAR1 activation triggers an inflammatory reaction of the tissue, often resulting in organ damage. Unfortunately, effective and/or specific therapeutic agents for these diseases are currently lacking. Additionally, rare congenital defects of IFNAR1 and its associated inflammatory response exist, for which specific drugs have not been yet available. On the other hand, inhibition of IFNAR1 affects the anti-tumor activity of the immune system in the presence of a malignant tumor.

The Tissue factor (TF)/Factor VIIa (FVlla) complex serves as the initiator of blood coagulation, leading to generation of thrombin (Ila), fibrin formation and receptor-mediated activation of platelets and other cells (Grover and Mackman, 2018; Huang et al., 1996; Mackman, 2004; Monkovic and Tracy, 1990; Narayanan, 1999). TF also contributes to other processes, such as tumor metastasis and angiogenesis, partially through its procoagulant function (Liu et al., 2019; Pawlinski et al., 2004; Rondon et al., 2019).

To the best knowledge of the inventors, an interaction between TF and IFNAR1 has not been suggested in the known art. A study by Manoharan et al. 2021 reveals a renoprotective function of TF, indicating that this protective function is linked to a suppression of the JAK / STAT pathway (Manoharan et al, "Podocyte Tissue Factor Suppresses Spontaneous Sterile Renal Inflammation"). However, this study does not disclose or suggests a connection between TF and IFNAR1.

The present invention is based on the surprising finding that TF binds to IFNAR1 and antagonizes its signaling, thereby preventing spontaneous sterile inflammation, and maintaining immune homeostasis. In other words, modulating the interaction between TF and IFNAR1 could potentially achieve both, an anti-inflammatory effect, i.e. for treating chronic inflammatory disease, autoimmune diseases or interferonopathy, and a pro-inflammatory effect, i.e. for treating a tumor disease. The use of pharmaceuticals, such as an immune modulator, that regulate the interaction of TF and IFNAR1 is plausible and beneficial for both conditions.

### SUMMARY OF THE INVENTION

In light of the prior art the technical problem underlying the present invention is to provide an IFNAR1 immune modulator for use as a medicament for regulating the interaction of TF and IFNAR1 in a subject.

This problem is solved by the features of the independent claims. Preferred embodiments of the present invention are provided by the dependent claims.

In a first embodiment the invention therefore relates to an interferon-a receptor (IFNAR1) immune modulator for use as a medicament for regulating the interaction of tissue factor (TF) and IFNAR1 in a subject.

It has been demonstrated for the first time that TF interacts with IFNAR1 and that IFNAR1 can be modulated in this process. This provides a novel approach to inhibit or activate signaling of IFNAR1. Since IFNAR1 has a function in inflammatory diseases and some malignant tumors, TF-mediated IFNAR1 modulation reveals a new therapeutic approach for inflammatory and oncological diseases. The use of modulators that regulate the interaction of TF and IFNAR1 is conceivable for both indication fields.

It was surprising that the loss of TF-IFNAR1 interaction can contribute to the severe thrombo-inflammatory response in Sars-CoV-19 infection. The embodiments of the invention are therefore promising staring points for developing compounds and treatments for Sars-CoV-19 infections and long covid. Therefore, it is preferred that the inflammatory diseases is a diseases associated with a thrombo-inflammatory response. It is preferred that the diseases is a Sars-CoV-19 infection.

The invention further relates to an IFNAR1 immune modulator for use as a medicament for treating chronic inflammatory disease, autoimmune diseases and/or interferonopathy in a subject by regulating the interaction of TF and IFNAR1.

Another aspect of the invention relates to an IFNAR1 immune modulator for use as a medicament for treating a tumor disease in a subject by regulating the interaction of TF and IFNAR1.

A further aspect of the invention relates to a method for treating chronic inflammatory disease, autoimmune diseases and/or interferonopathy in a subject by regulating the interaction of TF and IFNAR1.

A further aspect of the invention relates to a method for treating a tumor disease in a subject by regulating the interaction of TF and IFNAR1.

The various aspects of the invention are unified by, benefit from, are based on and/or are linked by the common and surprising finding that TF binds to IFNAR1 and antagonizes its signaling, preventing spontaneous sterile inflammation, and maintaining homeostasis.

The physiological role of TF, particularly during embryonic development or in the kidney, has remained unknown hitherto. However, the present invention, to the knowledge of the inventors, reveals that podocyte TF is required for renal immune-homeostasis in podocytes. Therefore, it is a surprising and beneficial finding that TF forms a heteromer with IFNAR1 restricting spontaneous IFNAR1 signaling and an associated sterile inflammatory response. The inhibitory function of TF towards IFNAR1 signaling is required for a normal glomerular and renal physiology. The unexpected finding unveils a previously unrecognized immunoregulatory receptor function of TF.

It has been demonstrated by the inventors that podocyte-specific TF deficient mice (TFΔPOD) have a phenotype similar to that in TFΔCT mice. Both mouse models show that TF maintains podocyte morphology, glomerular function, and renal immune homeostasis. Without being bound by the theory, it is suggested that the cytoplasmic TF-domain might contribute to the inhibition of IFNAR1-signaling in a TF-IFNAR1 heteromer.

Upon ligand binding, IFN-receptors undergo clustering, leading to transphosphorylation of receptor-associated Janus kinases (Babon et al., 2014). Without being bound by the theory, it is suggested that podocyte TF prevents spontaneous IFNAR1 clustering and signaling.

Coagulation and inflammation are closely linked and exhibit evolutionary commonality (Doolittle, 2011; Opal, 2000). Based on the surprising finding, the present invention for the first time enables modulation of immune homeostasis by regulating the interaction between TF and IFNAR1.

Until the present invention, an immune modulator targeting IFNAR1 has not been disclosed for use in the medical field. Therefore, in the present invention, the IFNAR1 immune modulator is used as a medicament for the first time and can be considered as a first medical use. This approach may involve novel patient groups and alternative ways to modulate the Jak / STAT pathways.

In embodiments, an IFNAR1 immune modulator is used as a medicament for treating chronic inflammatory disease, autoimmune diseases and/or interferonopathy in a subject by regulating the interaction ofTF and IFNAR1.

IFNAR1 is activated in the context of inflammatory diseases, including autoimmunological diseases, and leads to an inflammatory tissue response that usually results in organ damage. It was unexpected that the interaction of TF with IFNAR1 at the cell surface inhibits the IFNAR1-mediated effects of interferons (ligands of IFNAR1). As known in the art, there are also rare congenital defects of IFNAR1 and its dependent inflammatory response for which specific drugs are not yet available. Therefore, it is advantageous that the IFNAR1 immune modulator is able to treat chronic inflammatory disease, autoimmune disease and/or interferonopathy by regulating interaction of TF and IFNAR1.

In embodiments of the invention the subject has an interrupted interaction ofTF and IFNAR1. This occurs, in particular, in a subject suffering from chronic inflammatory disease, autoimmune diseases and/or interferonopathy. The overactive IFNAR1 downstream signaling, such as JAK/STAT pathway, can result in severe organ damage. The administration of the immune modulator according to the present invention can thus promote an anti-inflammatory process resulting in less organ damage due to an overactive immune reaction.

By using a genetic mouse model of TF-deficiency in podocytes the invention could identify a new molecular thrombo-inflammatory switch. TF-expression in podocytes is required for renal immune-homeostasis, which mechanistically depends on the interaction of TF with IFNAR1, preferred in a heteromer. It was shown that TF-IFNAR1 interaction prevents both TF-activity and IFNAR1 signaling. Upon stimulation, the TF-IFNAR1 interaction was lost, allowing (i) IFNAR1 signaling and (ii) TF to interact with fVll/fVlla, thus simultaneously promoting IFNR signaling and TF-/fVII-mediated coagulation activation and signaling, respectively. The invention enabled for the first time to utilize the immunoregulatory receptor function of TF.

Therefore, the administration of the modulator can promote anti-inflammatory processes. It is preferred that in these embodiments, the modulator binds to IFNAR1 so that the IFNAR1 signaling and TF mediated coagulation activity is suppressed.

It is also preferred that the administration of the modulator attenuates the IFNAR1/JAK/STAT signaling. The JAK-STAT signaling pathway is a chain of interactions between proteins in a cell, and is involved in processes such as immunity, cell division, cell death, and tumor formation. The pathway communicates information from chemical signals outside of a cell to the cell nucleus, resulting in the activation of genes through the process of transcription. There are three key elements of JAK-STAT signaling: Janus kinases (JAKs), signal transducer and activator of transcription proteins (STATs), and receptors (which bind the chemical signals). Given that many JAKs are associated with cytokine receptors, the JAK-STAT signaling pathway plays a major role in cytokine receptor signaling. Since cytokines are substances produced by immune cells that can alter the activity of neighboring cells, the effects of JAK-STAT signaling are often more highly seen in cells of the immune system.

The JAK/STAT pathway in cytokine receptor signaling can activate STATs, which can bind to DNA and allow the transcription of genes involved in immune cell division, survival, activation and recruitment. For example, STAT1 can enable the transcription of genes which inhibit cell division and stimulate inflammation. Also, STAT4 is able to activate NK cells (natural killer cells), and STATS can drive the formation of white blood cells. In response to cytokines, such as IL-4, JAK-STAT signaling is also able to stimulate STAT6, which can promote B-cell proliferation, immune cell survival, and the production of an antibody called IgE.

Therefore, it is advantageous that the administration of the modulator according to the invention attenuates the IFNAR1/JAK/STAT signaling and promote anti-inflammatory processes.

In preferred embodiments of the invention, the modulator is an IFNAR1 inhibitor/antagonist and binds the D2-D4 domains of IFNAR1.

It is a surprising finding that the interaction between TF and IFNAR1 is mediated by the interaction of the C-terminal fibronectin III domain of TF and D1-D4 domain of IFNAR1. This provides a putative structural model of the TF-IFNAR1 complex, in which preferably 31 amino acids (110 to 242 of TF) of TF interact with the D1-D4 domains of IFNAR1. Of these 31 amino acids, 29 are within the TF C-FNIII domain (138 to 242 amino acids of TF), which exhibits structural similarity with the interferon-α/β receptor domains (pfam09294). No interaction of the N-FNIII domain of TF with IFNAR1 was observed.

It is especially preferred that the modulator is a peptide or small medical compound.

In preferred embodiments, the modulator comprises at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31 of the amino acids selected from the list of Q110, G120, T121, K149, I152, W158, S160, S161, S162, S163, G164, K166, K169, T170, N171, T172, E174, L176, D178, V179, D180, K181, C186, V192, S195, P206, V207, E208, C209 and E213 of the tissue factor according to SEQ ID NO:1.

It is also preferred that the modulator comprises at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31 of the amino acids selected from the list of Q110, G120, T121, K149, I152, W158, S160, S161, S162, S163, G164, K166, K169, T170, N171, T172, E174, L176, D178, V179, D180, K181, C186, V192, S195, P206, V207, E208, C209 and E213 of the tissue factor according to SEQ ID NO:1, wherein the function of the modulator is maintained.

It is preferred that the modulator comprises 31 amino acids of the tissue factor (110-242 amino acids of TF) according to SEQ ID NO:1, wherein amino acids comprised in the modulator are preferred Q110, G120, T121, K149, I152, W158, S160, S161, S162, S163, G164, K166, K169, T170, N171, T172, E174, L176, D178, V179, D180, K181, C186, V192, S195, P206, V207, E208, C209 and E213 of SEQ ID NO: 1, or sequence having at least 70%, 75%, 80%, 85%, 90%, 95% sequence identity to SEQ ID NO: 1, wherein the function of the modulator is maintained.

It is further preferred that the modulator comprises 29 amino acids of tissue factor (138-242 amino acids of TF) according to SEQ ID NO:1, wherein critical amino acids are K149, I152, W158, S160, S161, S162, S163, G164, K166, K169, T170, N171, T172, E174, L176, D178, V179, D180, K181, C186, V192, S195, P206, V207, E208, C209 and E213 of SEQ ID NO: 1, or a sequence having at least 70%, 75%, 80%, 85%, 90%, 95% sequence identity to SEQ ID NO: 1, wherein the function of the modulator is maintained.

TF sequence is according to SEQ ID NO:1:

It is further preferred that the subject has a medical condition associated with renal dysfunction.

It is entirely unexpected that TF functions in restricting IFNAR1 signaling in renal glomeruli and thus as a rheostat of the glomerular micromilieu. This is in accordance with previous reports showing a role of IFNAR1/JAK/STAT signaling in renal diseases. In rodent models of glomerular injury (adriamycin, streptozotocin-induced hyperglycemia), JAK/STAT signaling is activated, and inhibition of JAK/STAT signaling reduces albuminuria and renal injury (Banes et al., 2004; Li et al., 2007). Likewise, genetic or antibody-mediate IFNAR1 inhibition reduces renal immunopathology in infectious (candida infection) and sterile (lupus-model) disease models (Baccala et al., 2012; Majer et al., 2012). While these studies established reduced leucocytes and granulocyte infiltration upon IFNAR1 genetic ablation in renal disease models, thus supporting a role of IFNAR1/JAK/STAT signaling in glomerular diseases, they did not identify mechanisms underlying increased glomerular JAK/STAT signaling (Li et al., 2007). In the present invention, based on the surprising finding that TF, whose expression is reduced in various glomerular diseases, antagonizes IFNAR1 signaling through a direct interaction, a new mechanism that induces IFNAR1/JAK/STAT signaling in glomerular diseases and (ii) a new function of TF as an immunoregulatory receptor have been identified.

In embodiments, the medical condition associated with renal dysfunction is a glomerular disease.

In embodiments, expression of TF is decreased in the glomerular disease.

In embodiments, the glomerular disease is associated with a reduced glomerular filtration rate (GFR), nephrotic syndrome and/or hypertension.

In embodiments, an IFNAR1 immune modulator is used as a medicament for treating a tumor disease in a subject by regulating the interaction of TF and IFNAR1.

In embodiments, the administration of the modulator promotes the immune response to the tumoric antigen.

The inhibition of IFNAR1 affects the anti-tumor activity of the immune system in the presence of a malignant tumor. Based on the surprising finding, a use of pharmaceuticals such as a IFNAR1 modulator that regulate the interaction of TF and IFNAR1 is conceivable for treating a tumor disease.

In embodiments, the modulator interrupts the interaction of TF and IFNAR1.

In embodiments, the modulator is an IFNAR1 agonist which interrupts the binding of TF to D2-D4 domains of IFNAR1.

Downregulation of IFNAR1 in tumor promotes an immunosuppressive environment that protects malignant cells from cytotoxic T lymphocytes (Katlinski et al., 2017). An IFNAR1 agonist can activate IFNAR1 signaling in malignant cells and promote anti-tumor activity of the immune system. It is preferred that the modulator comprises at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31 of the amino acids selected from the list ofQ110, G120, T121, K1-49, I152, W158, S160, S161, S162, S163, G164, K166, K169, T170, N171, T172, E174, L176, D178, V179, D180, K181, C186, V192, S195, P206, V207, E208, C209 and E213 of 110-242 amino acids of the tissue factor according to SEQ ID NO:1.

It is preferred that the modulator comprises at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31 of the amino acids selected from the list of Q110, G120, T121, K149, I152, W158, S160, S161, S162, S163, G164, K166, K169, T170, N171, T172, E174, L176, D178, V179, D180, K181, C186, V192, S195, P206, V207, E208, C209 and E213 of 110-242 amino acids of the tissue factor according to SEQ ID NO:1, wherein the function of the modulator is maintained.

It is further preferred that the modulator comprises 31 amino acids of tissue factor (110-242 amino acids of TF) according to SEQ ID NO:1, wherein amino acids comprised in the modulator are Q110, G120, T121, K149, I152, W158, S160, S161, S162, S163, G164, K166, K169, T170, N171, T172, E174, L176, D178, V179, D180, K181, C186, V192, S195, P206, V207, E208, C209 and E213 of SEQ ID NO: 1, or a sequence having at least 70%, 75%, 80%, 85%, 90%, 95% sequence identity to SEQ ID NO: 1, wherein the function of the modulator is maintained.

It is also preferred that the modulator comprises 29 amino acids of tissue factor (138-242 amino acids of TF) according to SEQ ID NO:1, wherein critical amino acids are K149, I152, W158, S160, S161, S162, S163, G164, K166, K169, T170, N171, T172, E174, L176, D178, V179, D180, K181, C186, V192, S195, P206, V207, E208, C209 and E213 of 110-242 amino acids of SEQ ID NO: 1, or a sequence having at least 70%, 75%, 80%, 85%, 90%, 95% sequence identity to SEQ ID NO: 1, wherein the function of the modulator is maintained.

It is preferred that the modulator binds to IFNAR1 and maintains the immune homeostasis.

The present invention is aimed at increasing the intensity of restoration of immune homeostasis, the violation of which is accompanied by a change in biological reactions and the development of an IFNAR1 immune modulator.

Without being bound by theory, the maintenance of immune homeostasis enables the bifunctional role of the immunomodulator promoting an anti-inflammatory process or promoting immune response to the tumoric antigen.

In further preferred embodiments, the modulator according to the invention is used in combination with a pharmaceutically acceptable carrier.

In embodiments, TF and IFNAR1 still form a heteromer, preferred a dimer, under the interrupted interaction. The modulator does not necessarily abolish the entire interaction between TF and IFNAR1. The modulator can just interrupt the interaction between TF and IFNAR1 to the extent that TF and IFNAR1 still form a heteromer. Advantageously, forming a heteromer between TF and IFNAR1 under the interrupted interaction contributes to the immune homeostasis.

In embodiments, TF and IFNAR1 do not interact physically under the interrupted interaction. The interaction between IF and IFNAR1 is completely abolished.

Embodiments and features of the invention described with respect to the IFNAR1 modulator for regulating the interaction of TF and IFNAR1, for treating chronic inflammatory disease, autoimmune disease and/or interferonopathy, treating a tumor disease in a subject, are considered to be disclosed with respect to each and every other aspect of the disclosure.

The various aspects of the invention are unified by, benefit from, are based on and/or are linked by the common and surprising finding that TF interacts with IFNAR1 and an IFNAR1 immune modulator can be used as a medicament for regulating the interaction of tissue factor (TF) and interferon-a receptor (IFNAR1) in a subject.

The invention utilizes a so far unknown dichotomous role of TF, with TF dampening IFN-signaling and thus inflammation in unstimulated cells but promoting inflammation in stimulated cells upon loss of the TF-IFNAR1 heteromer. The invention is also based on the finding that different results will be obtained depending on the time-point of analyses. Likewise, the efficacy of therapies targeting TF is dependent on the time-point of the intervention.

### DETAILED DESCRIPTION OF THE INVENTION

All cited documents of the patent and non-patent literature are hereby incorporated by reference in their entirety.

As used herein, the term "IFNAR1" refers to interferon- α receptor which is a subunit of IFN receptor. The IFN receptor is a heterodimer formed by IFNAR1, a 530 residue integral membrane protein, and IFNAR2, a 487 residue integral membrane protein. The extracellular portion of IFNAR1 is composed of four fibronectin type III repeats. IFNAR1 is located on chromosome 21 and OMIM numbers is 107450. IFNAR1 accession: AAH02590. The receptors are permanently associated with kinases belonging to the family Janus (JAK). The JAK/STAT pathway comprehends four members, JAK 1, 2, 3 and tyrosine kinase (Tyk) and seven Signal Transducer and Activation of Transcription (STAT 1-4, 5a, 5b and 6). The activation of IFNARs determines the phosphorylation of tyrosine residues on the receptor tail, this causes the recruitment of STAT1 and STAT2 through phosphorylated Tyk2 and JAK1. Upon binding with JAK1, STATs are phosphorylated by JAKs and are released as heterodimer of phosphorylated STAT1 and STAT2.

The sequence of IFNAR1 is according to the SEQ ID NO:2:

MMVVLLGATTLVLVAVAPWVLSAAAGGKNLKSPQKVEVDIIDDNFILRWNRSDESVGNVTFSFDY
QKTGMDNWIKLSGCQNITSTKCNFSSLKLNVYEEIKLRIRAEKENTSSWYEVDSFTPFRKAQIGP
PEVHLEAEDKAIVIHISPGTKDSVMWALDGLSFTYSLVIWKNSSGVEERIENIYSRHKIYKLSPETT
YCLKVKAALLTSWKIGVYSPVHCIKTTVENELPPPENIEVSVQNQNYVLKWDYTYANMTFQVQW
LHAFLKRNPGNHLYKWKQIPDCENVKTTQCVFPQNVFQKGIYLLRVQASDGNNTSFWSEEIKFD
TEIQAFLLPPVFNIRSLSDSFHIYIGAPKQSGNTPVIQDYPLIYE IIFWENTSNAERKKKKK

As used herein, the term "TF" refers to tissue factor, which is a transmembrane receptor for Factor Vll/Vlla (FVII/Vlla). It is constitutively expressed by cells surrounding blood vessels. The endothelium physically separates this potent "activator" from its circulating ligand FVII/FVlla and prevents inappropriate activation of the clotting cascade. TF is also expressed in certain tissues, such as the heart and brain, and provides additional hemostatic protection to these tissues. Small amounts of TF are also present in blood in the form of microparticles, which are small membrane vesicles derived from activated and apoptotic cells. Levels of microparticle TF increase in a variety of diseases, such as sepsis and cancer. Recombinant FVlla has been developed as an effective hemostatic drug for the treatment of hemophilia patients with inhibitory antibodies.

As used herein, the term "interaction of TF and IFNAR1" shall mean the physical contact between TF and IFNAR1. The interaction ofTF and INFAR1 on the cell surface inhibits the IFNAR1-mediated effects of interferons, which is the ligand of IFNAR1. INFNAR1 has various effects. In embodiments, IFNAR1 is activated in inflammatory diseases, including autoimmunological diseases, and leads to an inflammatory reaction of the tissue, which usually leads to organ damages. In embodiments, the inhibition of IFNAR1 by the interaction between TF and IFNAR1 affects the anti-tumor activity of the immune system in the presence of a malignant tumor.

Protein-protein-interaction can be determined by different high-throughput experimental and computational methods known in the art, such as yeast two-hybrid technique, tandem affinity purification-MS, co-immunoprecipitation, nanotemper, domain-pairs-based approach for predicting the interactions between proteins by the domain-domain interactions or structurebased approach predicting protein-protein interaction based on the structural similarity. In the context of the present invention, the interaction of TF and IFNAR1 has been demonstrated by coimmunoprecipitation wherein IFNAR1 was detected in TF-pulldown. The direct interaction between the two proteins was demonstrated by a nanotemper experiment.

The amino acids in TF, which are required for the interaction, can be identified by different methods known in the art, such as Rosetta protein-protein docking analysis and confirmed by site-direct mutagenesis.

As used herein, the term "immune modulator" shall mean a substance that stimulates or suppresses the immune system and may help the body to fight against cancer, infections or other disease. An immune modulator can be an antibody, preferably monoclonal, a cytokine, a vaccine, a peptide or a compound which affect specific parts of the immune system. In embodiments, an immune modulator can adjust the immune response to the correct level, strengthen weak immune systems and control overactive immune system. In embodiments of the present invention, an IFNAR1 immune modulator which interrupts the interaction between TF and IFNAR1 partially or completely, can adjust the downstream signaling pathway JAK/STAT and correct the immune system.

There are various methods in the art for identifying or constructing an IFNAR1 immune modulator. IFNAR1 modulator peptides are designed based on crucial TF amino acids that are e.g. identified by Rosetta protein-protein docking analysis and confirmed by site-directed mutagenesis. In embodiments, after identifying the required amino acids residues in TF, potential peptides that mimic TF interaction with IFNAR1 are screened using phage display technology and their relevance will be confirmed by in vitro experiments. In other embodiments, potential peptides may be synthesized after identifying the required amino acids residues. These potential peptides are used for preclinical studies using mouse models of cancer, inflammatory and autoimmune disease. Peptides generally have high specificity, good efficacy and better membrane permeability. In alternative embodiments, a compound library can be used for searching small-chemical compounds, which have the same effect as a peptide. These compounds are identified using interferon-stimulated response element (ISRE)-luciferase reporter assay. Small-chemical compounds are generally easier to produce, show better pharmacokinetics and could be better modified, e.g. regarding stability or bioavailability, compared to peptides.

As used herein, the term "antagonist" shall mean a receptor ligand or substance that blocks or dampens a biological response by binding to and blocking a receptor rather than activating it like an agonist. Antagonist drugs interfere in the natural operation of receptor proteins. They are sometimes called blockers; examples include alpha blockers, beta blockers, and calcium channel blockers. Antagonists have affinity but no efficacy for their cognate receptors, and binding will disrupt the interaction and inhibit the function of an agonist or inverse agonist at receptors. Antagonists mediate their effects by binding to the active site or to the allosteric site on a receptor, or they may interact at unique binding sites not normally involved in the biological regulation of the receptor's activity. Antagonist activity may be reversible or irreversible depending on the longevity of the antagonist-receptor complex, which, in turn, depends on the nature of antagonist-receptor binding. The majority of drug antagonists achieve their potency by competing with endogenous ligands or substrates at structurally defined binding sites on receptors. In embodiments, the IFNAR1 immune modulator can sequester IFNAR1 blocking the downstream process, in particular, the JAK/STAT mediated immune response.

As used herein, the term "agonist" shall mean a chemical that activates a receptor to produce a biological response. Receptors are cellular proteins whose activation causes the cell to modify what it is currently doing. In embodiments, the present invention relates to an IFNAR1 immune modulator which functions as an IFNAR1 agonist activating the downstream immune response to tumoric antigen.

As used herein, the term "chronic inflammatory disease" refers to slow, long-term inflammation lasting for prolonged periods of several months to years. The extent and effects of chronic inflammation vary with the cause of the injury and the ability of the body to repair and over the damage. Chronic inflammatory disease are, for example, rheumatoid arthritis, systemic lupus erythematosus and multiple sclerosis.

As used herein, the term "autoimmune disease" shall mean a condition that results from an anomalous response of the immune system, wherein it mistakenly targets and attacks healthy, functioning parts of the body as if they were foreign organisms. Autoimmune diseases include but are not limited to celiac disease, type 1 diabetes, Graves' disease, inflammatory bowel diseases (such as Crohn's disease and ulcerative colitis), multiple sclerosis, alopecia areata, Addison's disease, pernicious anemia, psoriasis, rheumatoid arthritis, and systemic lupus erythematosus.

As used herein, the term "interferonopathy" shall mean the medical condition associated with overproduction of Type I interferons, such as IFN-α, IFN-β and IFN-Ω. Interferonopathies are a group of inherited autoinflammatory diseases, characterized by a dysregulation of the interferon pathway, leading to constitutive upregulation of its activation mechanisms or downregulation of negative regulatory systems.

As used herein, the term "tumor" shall mean neoplasm. This term includes solid and hematological tumors.

Examples of hematological tumors include, but are not limited to: leukemias, including acute leukemias (such as acute lymphocytic leukemia, acute myelocytic leukemia, acute myelogenous leukemia and myeloblastic, promyelocytic, myelomonocytic, monocytic and erythroleukemia), chronic leukemias (such as chronic myelogenous leukemia, and chronic lymphocytic leukemia), myelodysplastic syndrome, and myelodysplasia, polycythemia vera, lymphoma, (such as Hodgkin's disease, all forms of non-Hodgkin's lymphoma), multiple myeloma, Waldenstrom's macroglobulinemia, and heavy chain disease.

Examples of solid tumors, such as sarcomas and carcinomas, include, but are not limited to: fibrosarcoma, myxosarcoma, liposarcoma, chondrosarcoma, osteogenic sarcoma, and other sarcomas, synovioma, mesothelioma, Ewing's tumor, leiomyosarcoma, rhabdomyosarcoma, colon carcinoma, pancreatic cancer, breast cancer, lung cancer, ovarian cancer, prostate cancer, hepatocellular carcinoma, squamous cell carcinoma, basal cell carcinoma, adenocarcinoma, sweat gland carcinoma, sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, bladder carcinoma, melanoma, and CNS tumors (such as a glioma, astrocytoma, medulloblastoma, craniopharyogioma, ependymoma, pinealoma, hemangioblastoma, acoustic neuroma, oligodendroglioma, menangioma, meningioma, neuroblastoma and retinoblastoma).

As used herein, the term "tumoric antigen" shall mean a tumor antigen which can stimulate tumorspecific T cell-defined immune responses or antibodies to tumor cells.

As used herein, the term "interrupted interaction" shall mean that the physical interaction between two proteins are partially or completely abolished. If the interaction is partially interrupted, the function of the heteromer can be partially or completely abolished. In the context of the present invention, an IFNAR1 immune modulator can function as an inhibitor / antagonist to sequester the IFNAR1 so that the overactive downstream immune response is controlled. Further, an IFNAR1 immune modulator can function as an IFNAR1 agonist which interrupts the interaction of TF and IFNAR1 and promote the immune response to the tumoric antigen.

As used herein, the term "anti-inflammatory process" shall mean a process that reduces inflammation. In embodiments, the IFNAR1 immune modulator blocks IFNAR1/JAK/STAT signaling pathway that cause overactive inflammation.

As used herein, the term "promote" the anti-inflammatory process in the context of the invention shall mean enhance, stimulate, increase the anti-inflammatory process.

As used herein, the term "medical condition associated with renal dysfunction" shall mean any medical condition associated with renal dysfunction. Renal dysfunction, also called renal failure, is a condition in which the kidneys stop working and are not able to remove the waste and extra fluid from the blood or keep the body chemicals in balance. Renal dysfunction can affect for example the function of electrolyte and volume regulation, excretion of nitrogenous waster, elimination of exogenous molecule, synthesis of a variety of hormones or metabolism of low molecular weight proteins. In embodiments of the invention, the medical condition associated with renal dysfunction is an acute renal failure. It is also preferred, that the medical condition associated with renal dysfunction is chronic renal failure.

As used herein, the term "a glomerular disease" shall mean a condition that can damage kidneys. The disease attacks tiny filters in the kidneys, called glomeruli, where the blood is cleaned. Damaged glomeruli can allow proteins and sometimes red blood cells to leak into the urine. One of the proteins in the blood is albumin. If too much albumin leaks into the urine, fluid can build up in the body, leading to swelling in face, hands, feet, or legs. In some cases, glomerular disease can also prevent the kidneys from properly removing waste products, causing wastes to build up in the blood.

As used herein, the term "immune homeostasis" shall mean the delicate and finely regulated balance of appropriate immune activation and suppression in tissues and organs, driven by a myriad of cellular players and chemical factors.

The present invention relates further to pharmaceutically acceptable salts of the immune modulator described herein. The term "pharmaceutically acceptable salt" refers to salts or derivatives of the compounds described herein prepared by conventional means that include basic salts of inorganic and organic acids. "Pharmaceutically acceptable salts" are also inclusive of the free acid, base, and zwitterionic forms. Descriptions of suitable pharmaceutically acceptable salts can be found in Handbook of Pharmaceutical Salts, Properties, Selection and Use, Wiley VCH (2002).

For therapeutic use, salts of the immune modulator are those wherein the counter-ion is pharmaceutically acceptable. However, salts of acids and bases which are non-pharmaceutically acceptable may also find use, for example, in the preparation or purification of a pharmaceutically acceptable compound.

The term "treatment" or "therapy" refers to administering an active agent with the purpose to cure, heal, alleviate, relieve, alter, remedy, ameliorate, improve, or affect a condition (e.g., a disease), the symptoms of the condition, or to prevent or delay the onset of the symptoms, complications, biochemical indicia of a disease, or otherwise arrest or inhibit further development of the disease, condition, or disorder in a statistically significant manner.

Sequence disclosed in the present invention:

| **SEQ ID NO:** | **Sequence** | **Description** |
|---|---|---|
| 1 | | TF sequence |
| 2 | MMVVLLGATTLVLVAVAPWVLSAAAGGKNLKSPQKVEVDIIDD NFILRWNRSDESVGNVTFSFDYQKTGMDNWIKLSGCQNITST KCNFSSLKLNVYEEIKLRIRAEKENTSSWYEVDSFTPFRKAQIG PPEVHLEAEDKAIVIHISPGTKDSVMWALDGLSFTYSLVIWKNS SGVEERIENIYSRHKIYKLSPETTYCLKVKAALLTSWKIGVYSPV HCIKTTVENELPPPENIEVSVQNQNYVLKWDYTYANMTFQVQ WLHAFLKRNPGNHLYKWKQIPDCENVKTTQCVFPQNVFQKGI YLLRVQASDGNNTSFWSEEIKFDTEIQAFLLPPVFNIRSLSDSF HIYIGAPKQSGNTPVIQDYPLIYE IIFWENTSNAERKKKKK | IFNAR1 sequence |

### FIGURES

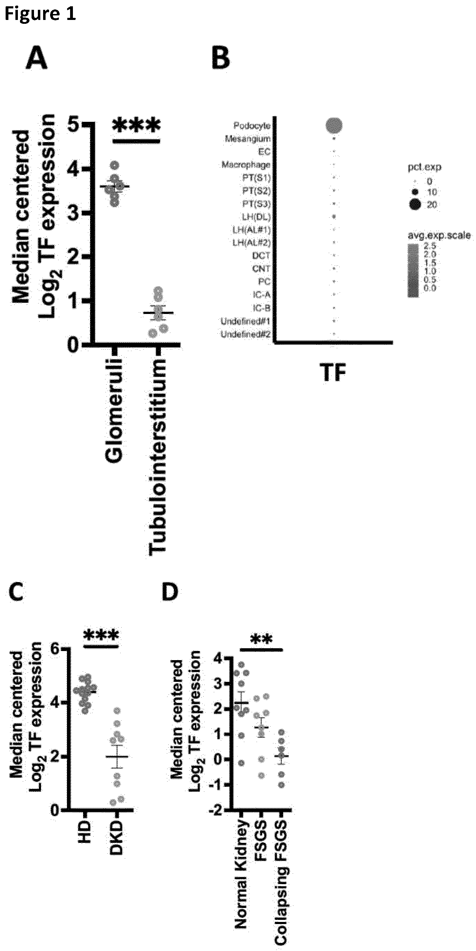

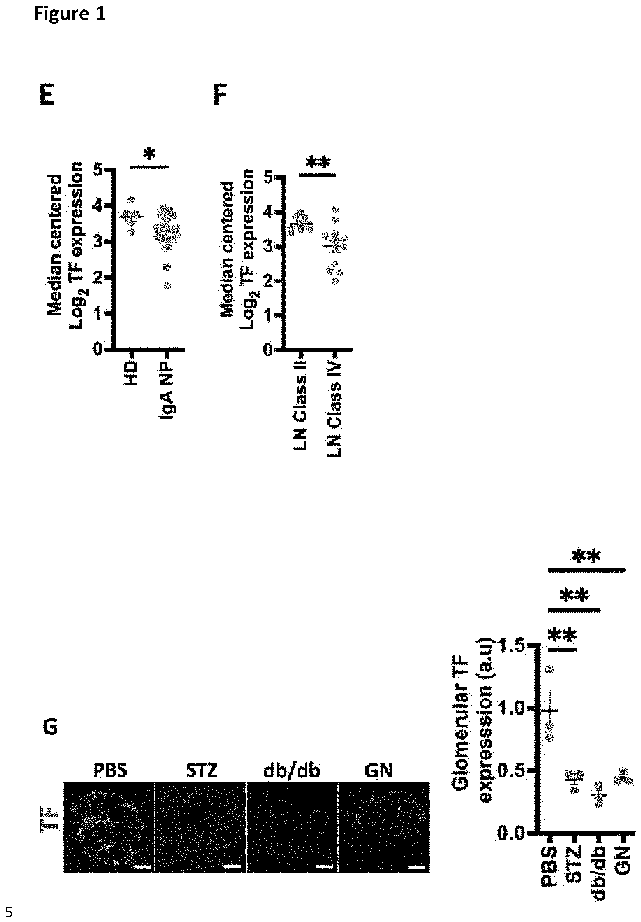

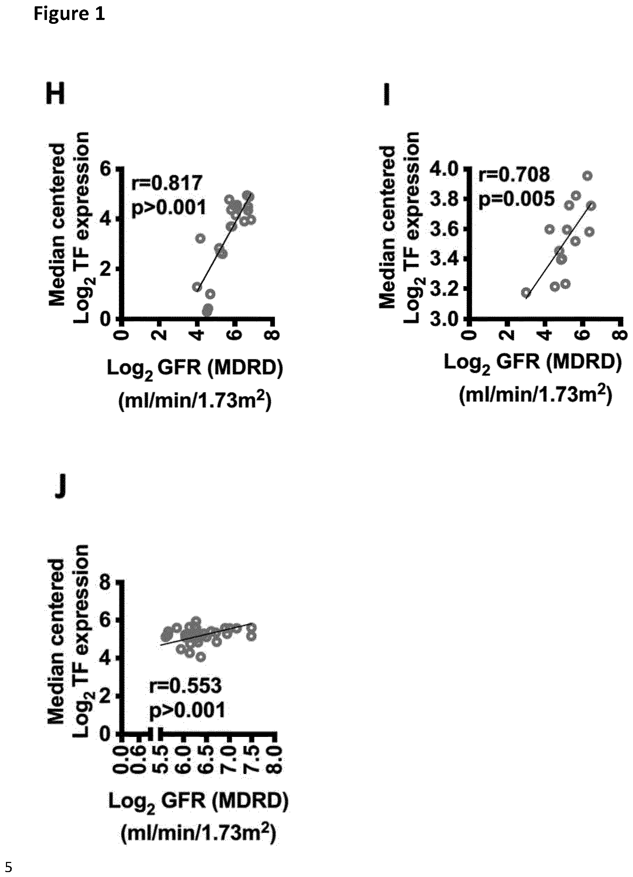

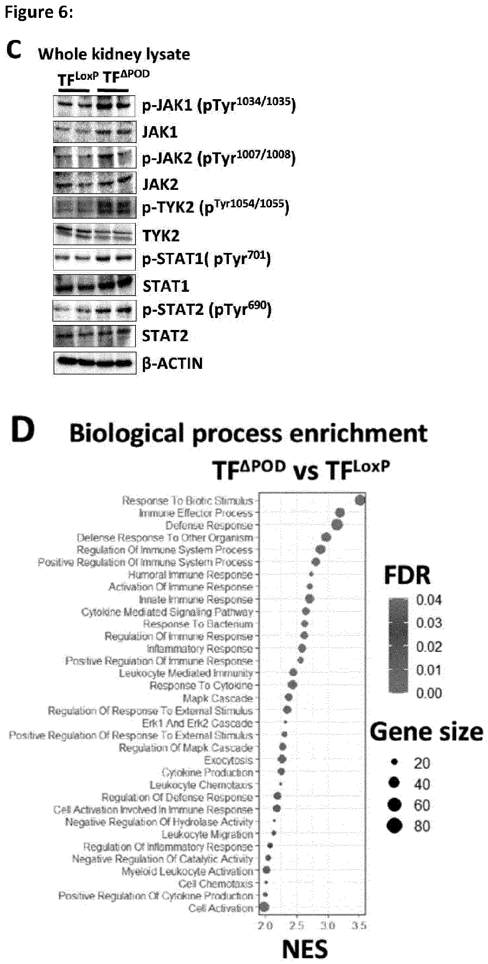

**Figure 1** **shows differential expression of glomerular TF in renal diseases.**
   **(A)** Dot plot representing TF mRNA expression in the glomeruli and tubulointerstitum of healthy human kidney. Data obtained from the Lindenmeyer microarray dataset (Nephroseq database). **(B)** Dot plot representing TF mRNA expression from single cell transcriptomics of healthy human adult kidney from Kidney Interactive Transcriptomics database (Wu and Uchimura et al, Cell Stem Cell 2018). **(C-E)** Dot plot representing glomerular TF mRNA expression in healthy donors (HD) compared to patients with diabetic kidney disease (DKD; Woroneicka diabetic glomeruli microarray dataset, C), with focal segmental sclerosis (FSGS) or collapsing FSGS (Hodgin FSGS glomeruli microarray dataset, D), or with IgA nephropathy (IgA NP, Reich IgAN glomeruli microarray dataset, E). **(F)** Dot plot representing glomerular TF expression from lupus nephritis (LN) class II and LN class IV (Berthier lupus nephritis microarray dataset) patient's datasets. All datasets obtained from the Nephroseq database. Dot plots summarizing data (mean ± SEM; each dot represents one donor). **(G)** Representative images of glomerular TF expression in mice exposed to PBS, streptozotocin (STZ), glomerulonephritis (GN) or in db/db mice. Dot plot with mean ± SEM summarizing data for glomerular TF staining in arbitrary units (a.u.). Each dot represents one mice. *P* values determined by one-way ANOVA with Tukey-*post* hoc comparison (***P*<0.002). (H-**J)** Correlation analyses of glomerular TF mRNA expression and glomerular filtration rate (GFR) in diabetic patients (H), in patients with nephrotic syndrome (I), and patients with hypertensive nephropathy (J). Data obtained from the Nephroseq database (G: Woroneicka diabetic glomeruli; H: Sampson nephrotic syndrome glomeruli dataset; I: Neusser hypertension glomeruli dataset). Each dot represent data from one donor. Confidence interval of r (Pearson coefficient) and *P* values were calculated by linear regression; **P*<0.05. (**r*<0.05). **A, C-F:** *P* values determined by two-tailed Student's *t* test (**P*<0.05, ***P*<0.002, ****P*<0.001).

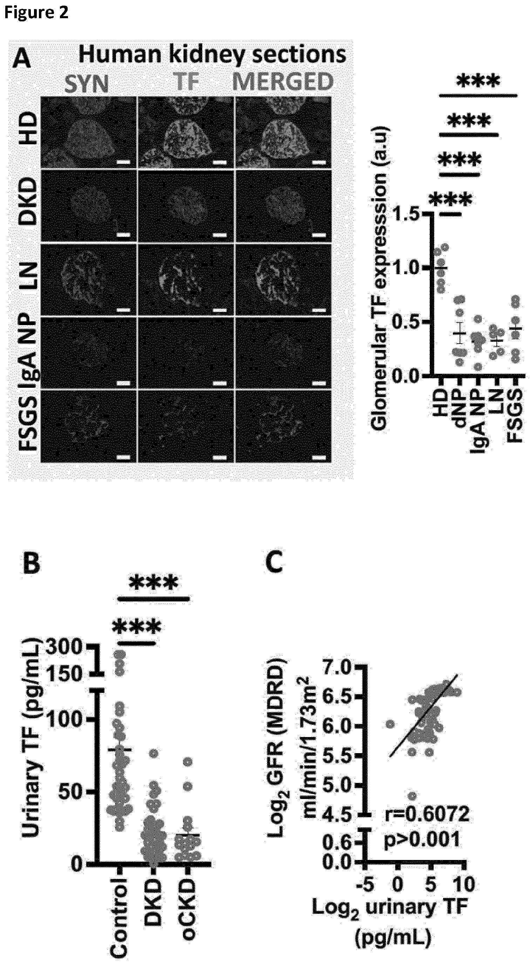

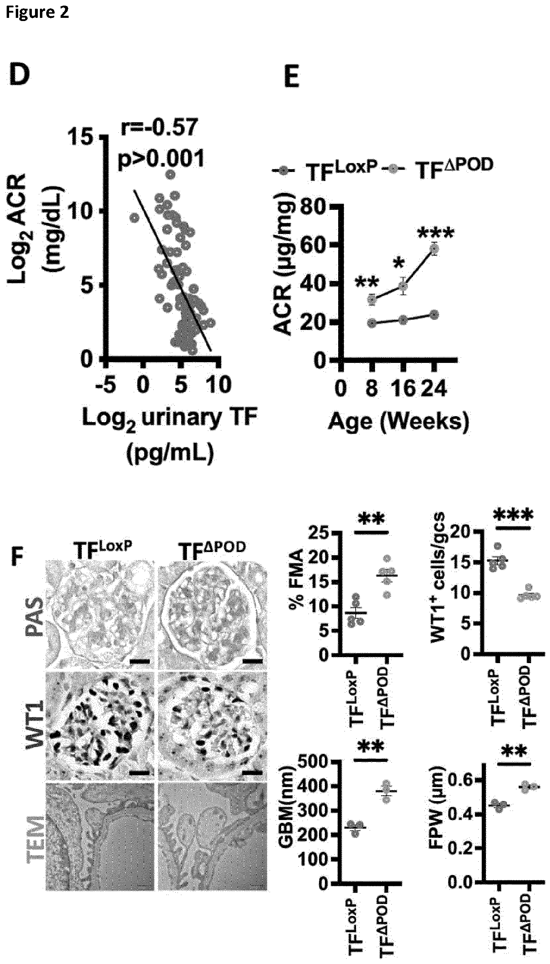

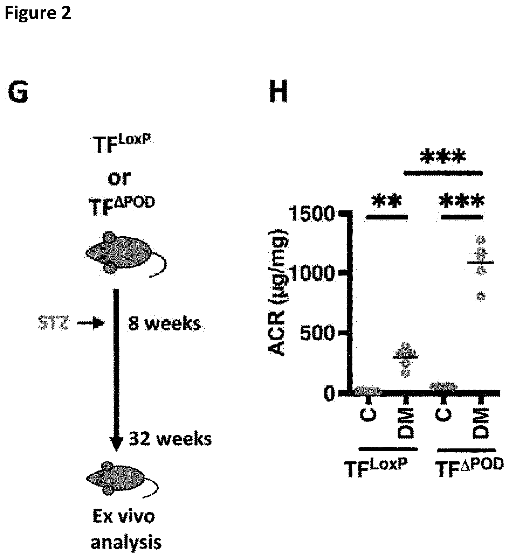

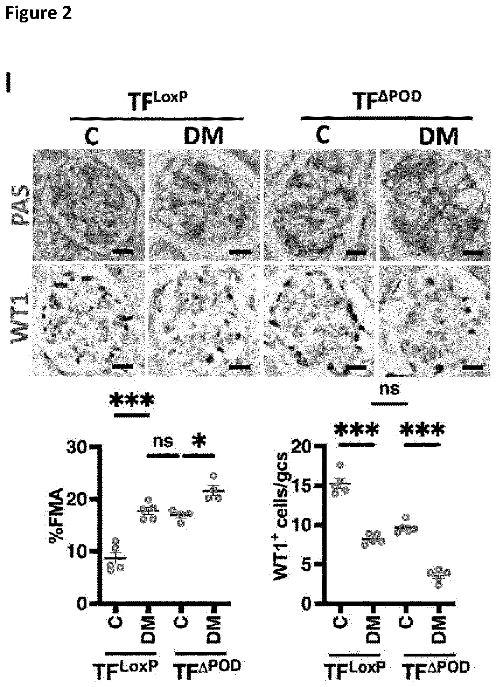

**Figure 2** **shows podocyte TF loss induced glomerulopathy.**
   **(A)** Representative images of glomerular TF expression from the healthy donor (HD) or patients with diabetic kidney disease (DKD), focal segmental glomerulosclerosis (FSGS), lupus nephritis (LN), or IgA nephropathy (IgA NP);staining for TF (middle lane), and synaptopodin (SYN;left lane); scale bar: 50 µm. Dot plot represents the quantification of glomerular TF expression in different kidney diseases. Dot plot summarizing mean ± standard error mean (SEM) of at least 5 human samples per group. **(B)** Dot plot represents the quantification of urinary TF (uTF) levels from kidney disease free patients (Control; n = 38) or diabetic kidney disease (DKD; n = 33) or other chronic kidney diseases (oCKD; n =15) that includes polycystic kidney disease, LN, and IgA NP. Dot plot summarizing mean ± SEM. Each dot represents data from one patient. **(C)** Correlation analyses of uTF and glomerular filtration rate (GFR) in control and DKD patients. **(D)** Correlation analyses of uTF and urine albumin-creatinine ratio (ACR) in control and DKD patients. **(E)** Line graph (mean ± SEM) summarizing urine albumin creatinine ratio (ACR) from TF^{LoxP} and TF^{ΔPOD} mice at different age. **(F)** Representative images of PAS-stained sections, immunohistochemical staining for WT1, and of TEM images obtained from mouse glomeruli of TF^{LoxP} and TF^{ΔPOD} mice. Dot plot summarizing data (mean ± SEM; each dot represents one mouse) for fractional mesangial area (FMA), foot process width (FPW), glomerular basement membrane (GBM) thickness, and WT1 positive cells per glomerular cross sections (gcs; at least 10 gcs per mice were analyzed); scale bar: 20 µm. **(G)** Schematic illustration of streptozotocin (STZ)-induced hyperglycemia in mice. **(H, I)** Dot plot representing urine albumin-creatinine-ratio (ACR, C) levels and representative glomerular images of glomerular sections (D) with PAS-stain or immunohistochemical WT1 staining obtained from 24 weeks diabetic (DM) or control (C) TF^{LoxP} and TF^{ΔPOD} mice. Dot plot with mean ± SEM summarizing data for ACR (C), FMA (fractional mesangial area (FMA, in percent), and WT1⁺ cells per glomerular cross sections (gcs; at least 10 gcs per mice were analyzed, F); each dot represent data from one mouse; scale bar: 20 µm. **A, B:** *P* values determined using one-way ANOVA with Dunnet-*post* hoc comparison (****P*<0.001). **C, D:** Confidence interval of r (Pearson coefficient) and *P* values were calculated by linear regression; **P*<0.05. (**r*<0.05). **E, F:** *P* values determined by two-tailed Student's *t* test (**P*<0.05, ***P*<0.002, ****P*<0.001). **H**, **I**: *P* values determined by two-way ANOVA with Tukey-post hoc comparison (**P*<0.05, ***P*<0.002, ****P*<0.001, ns- not significant).
**Figure 3** **shows loss of podocyte TF aggravates diabetic nephropathy.**
   **(A)** Scheme representing the generation of podocyte-specific TF knockout mice (TF^{ΔPOD}). **(B)** Representative images of mouse glomeruli stained for TF (middle row) and SYN (top row) in kidney sections of TF^{LoxP} and TF^{ΔPOD} mice; scale bar: 20 µm.
**Figure 4** **shows loss of podocyte TF does not impair intrauterine kidney development.**
   Representative images of WT1 immunohistochemical staining of mouse kidney sections obtained from TF^{LoxP} and TF^{ΔPOD} mice on post-natal day1; scale bar: 200 µm. Dot plot summarizing data (mean ± SEM; each dot represents data from one mouse) of the number of vesicle bodies, S-shaped bodies, glomeruli and WT1 positive cells per glomerular cross sections (gcs; at least 10 gcs per mice were analyzed from 5 litters in each group) in kidneys of TF^{LoxP} and TF^{ΔPOD} mice on post-natal day 1. *P* values determined by two-tailed Student's *t* test (**P*<0.05, ***P*<0.002, ****P*<0.001).

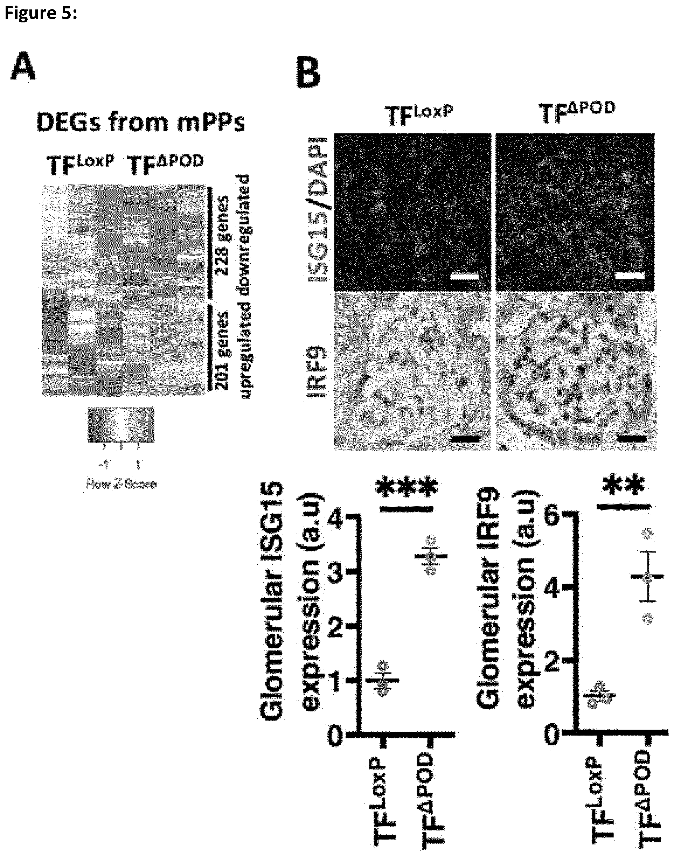

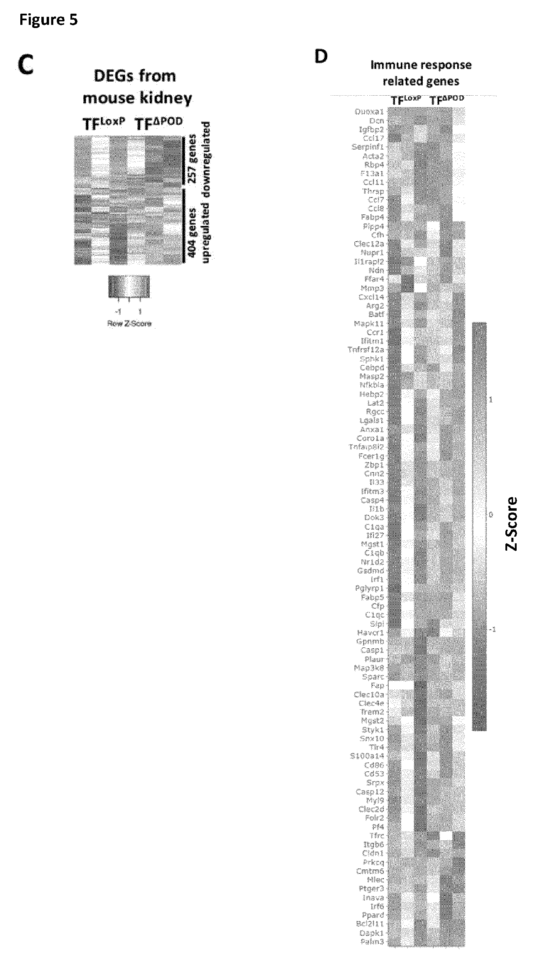

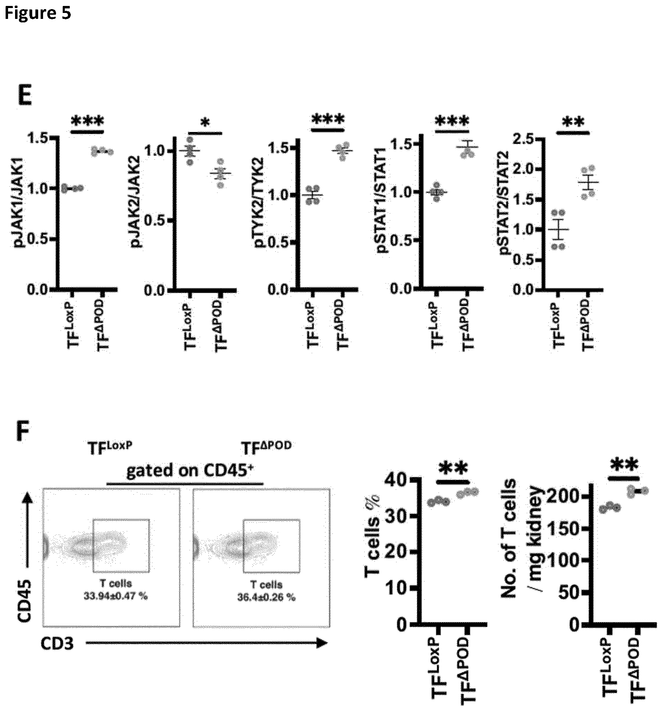

**Figure 5** **shows podocytes TF loss induced disruption of renal immune homeostasis.**
   **(A)** Heatmap representing differentially expressed genes (DEGs) in mPPs of TF^{LoxP} and TF^{ΔPOD} mice. Three samples per genotype,Z-score represents up and downregulation of genes. **(B)** Representative images of glomerular ISG15 and IRF9 expression from TF^{LoxP} and TF^{ΔPOD} mice. Dot plot with mean ± SEM summarizing data for glomerular ISG15 and IRF9 staining in arbitrary units (a.u: at least 10 gcs per mice were analyzed). **(C)** Heatmap representing DEGs in whole kidney transcriptomes of TF^{LoxP} and TF^{ΔPOD} mice. Three samples per genotype, Z-score represents up and down regulation of genes **(D)** Heatmap representing the immune response related genes from whole kidney transcriptomes of TF^{LoxP} and TF^{ΔPOD} mice. Three samples per genotype, Z-score represents up and downregulation of genes. **(E)** Dot plots summarizing data (mean ± SEM; each dot represents data from one mouse) of immunoblots quantification corresponding to Figure 6C. *P* values determined by two-tailed Student's *t* test (**P*<0.05, ****P*<0.001). **(F)** FACS immunophenotyping of T cells (CD45⁺CD3⁺, in whole kidneys of TF^{LoxP} and TF^{ΔPOD}. Dot plot summarizing data (mean ± SEM; each dot represents data from one mouse). *P* values were determined using a two-tailed Student's *t* test (**P*<*0.002).

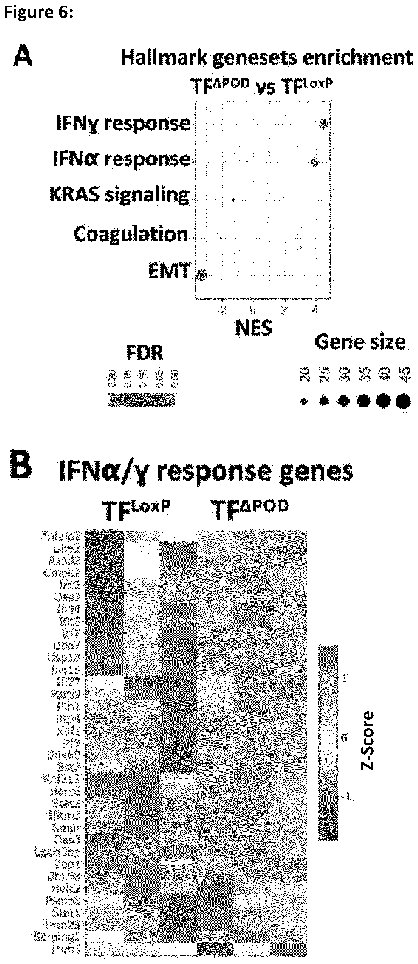

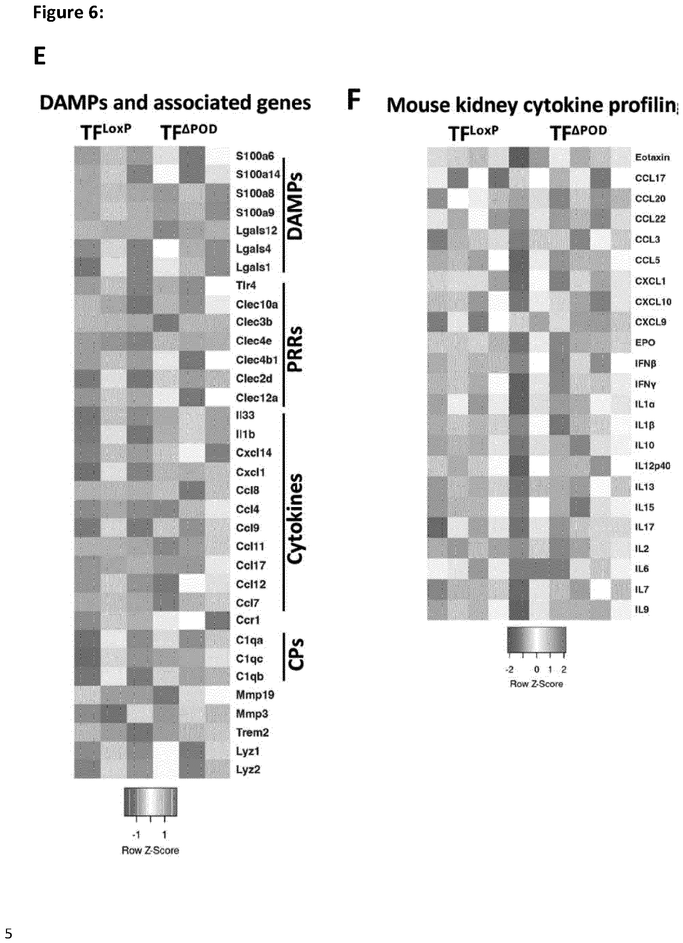

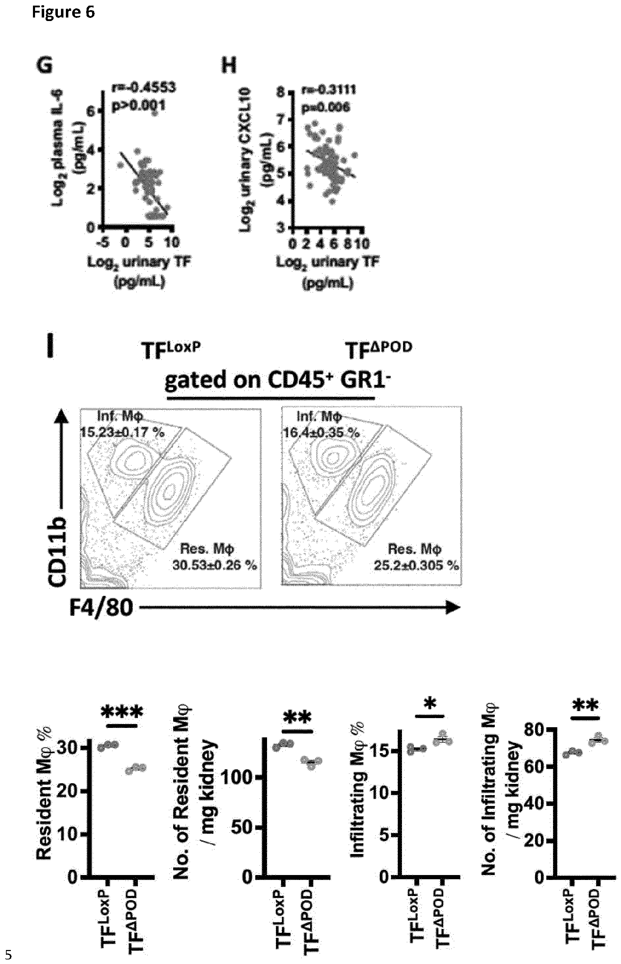

**Figure 6** **shows podocyte TF loss induced interferon response in mouse primary podocytes (mPPs) causing sterile renal inflammation and disrupted immune homeostasis.**
   **(A)** Dot plot summarizing genome sequence enrichment analysis (GSEA) of hallmark gene sets enriched in TF^{ΔPOD} compared to TF^{LoxP} mouse primary podocytes (mPPs), represented as normalized enrichment score (NES). **(B)** Heatmap represents IFNa/y response genes from the transcriptome analysis in mPP of TF^{LoxP} and TF^{ΔPOD} mice. Three samples per genotype, Z-score represents up and down regulation of genes **(C)** Representative immunoblots image of p-JAK1, JAK1, p-JAK2, JAK2, p-TYK2, TYK2, p-STAT1, STAT1, p-STAT2, STAT2, β-ACTIN of whole kidney protein lysates from 16 weeks old TF^{LoxP} and TF^{ΔPOD} mice. For each phosphorylated protein the non-phosphorylated form is shown for normalization. β-ACTIN serves as a loading control. **(D)** Dot plot shows positively enriched biological processes obtained by GSEA in whole kidney transcriptomes of TF^{ΔPOD} *versus* TF^{LoxP} mice, ordered and represented by the normalized enrichment score (NES). **(E)** Heatmap representing DAMPs and associated genes expression from whole kidney transcriptomes of TF^{LoxP} and TF^{ΔPOD} mice. Three samples per genotype,Z-score represents up and down regulation of genes. **(F)** Heatmap representing the significantly altered (**P*<0.05; two-tailed Student's *t* test) cytokines from mouse 44-plex cytokine profiling of whole kidney lysates from 24 weeks old TF^{LoxP} and TF^{ΔPOD} mice (5 samples per genotype). Z-score represents up and down regulation of genes. **(G)** Correlation analyses of uTF and plasma IL-6 levels in control and DKD patients. **(H)** Correlation analyses of uTF and urinary CXCL10 levels in control and DKD patients. **(I)** FACS immunophenotyping of infiltrating (F4/80^{low}CD11b^{hi}) and resident (F4/80^{hi}CD11b^{low}) macrophages in whole kidneys of TF^{LoxP} and TF^{ΔPOD} mice. Dot plot summarizing data (mean ± SEM; each dot represents data from one mouse). *P* values determined by two-tailed Student's *t* test (**P*<0.05, ****P*<0.001). **G, H:** Confidence interval of r (Pearson coefficient) and *P* values were calculated by linear regression; *P<0.05. (**r*<0.05).

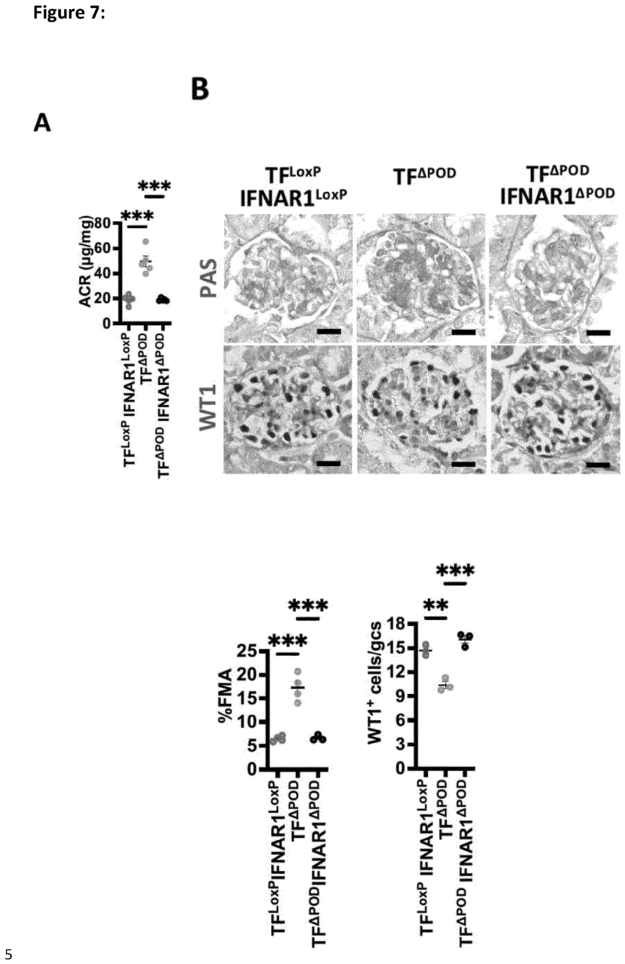

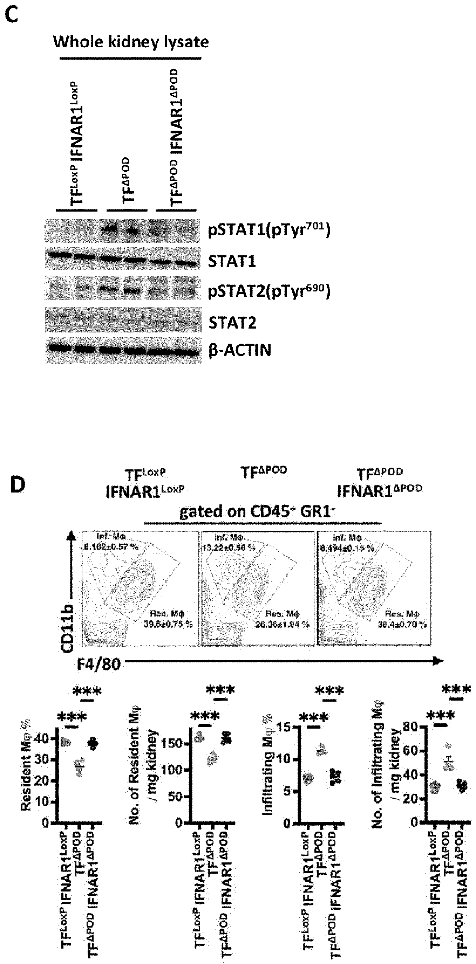

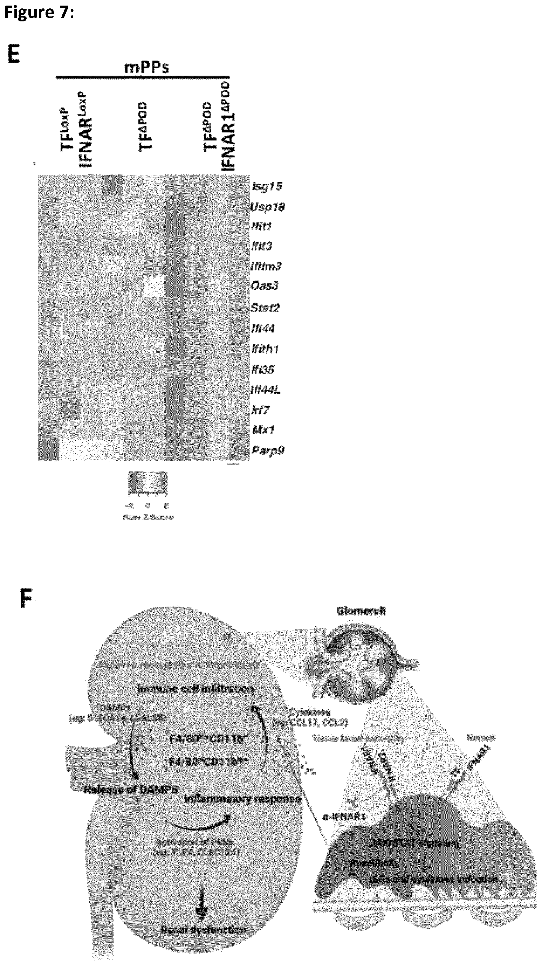

**Figure 7** **shows reversal of renal injury in mice lacking TF and IFNAR1 in podocytes.**
   **(A,B)** Dot plot (A) representing urine albumin-creatinine-ratio (ACR) levels and representative images (B) of PAS-stained (top) or WT1 stained (bottom, immunohistochemistry) glomerular sections from 16 weeks old TF^{LoxP}IFNAR1^{LoxP} (control), TF^{ΔPOD} and TF^{ΔPOD} IFNAR1^{ΔPOD} mice. Dot plot with mean ± SEM summarizing data for ACR (A), WT1⁺ cells per glomerular cross sections (gcs; at least 10 gcs per mice were analyzed), or FMA (fractional mesangial area) in percent; each dot represent data from one mouse; scale bar: 20 µm. **(C)** Representative immunoblot images of p-STAT1, STAT1, p-STAT2, STAT2, β-ACTIN in whole kidney protein lysates from 16 weeks old TF^{LoxP}IFNAR1^{LoxP} ,TF^{ΔPOD} and TF^{ΔPOD}IFNAR1^{ΔPOD} mice. For each phosphorylated protein the non-phosphorylated form is shown for normalization. β-ACTIN serves as loading control. **(D)** FACS immunophenotyping of infiltrating (F4/80^{low}CD11b^{hi}) and resident (F4/80^{hi}CD11b^{low}) macrophages in whole kidneys of 16 weeks old TF^{LoxP}IFNAR1^{LoxP}, TF^{ΔPOD} and TF^{ΔPOD} IFNAR1^{ΔPOD} mice. Dot plot summarizing data (mean ± SEM; each dot represents data from one mouse). **(E)** Heatmap representing significantly altered (**P*<0.05; one-way ANOVA) mRNA expression of selected ISGs in mouse primary podocytes (mPP) isolated from TF^{LoxP}IFNAR1^{LoxP}, TF^{ΔPOD} and TF^{ΔPOD}IFNAR1^{ΔPOD} mice. 18s RNA level were used for normalization. Z-score represents up and down regulation of genes. **(F)** Scheme summarizing proposed model: TF binds to IFNAR1, thus dampening IFNAR1/JAK/STAT signaling. In the absence of TF, increased IFNAR1/JAK/STAT signaling causes sterile inflammation and disrupts organ immune homeostasis. **A, B, D:** *P* values calculated by one-way ANOVA with Tukey-*post hoc* comparison (***P*<0.002, ****P*<0.001).

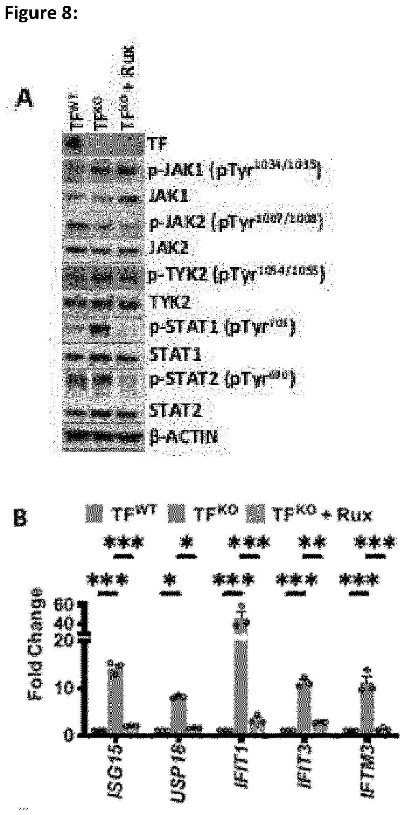

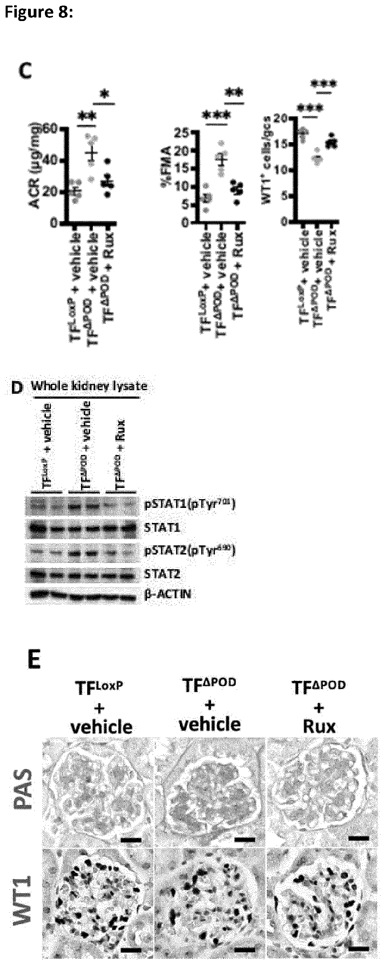

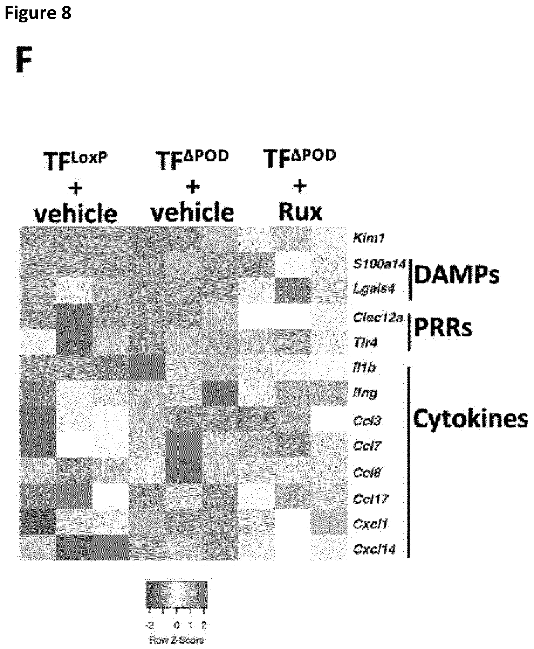

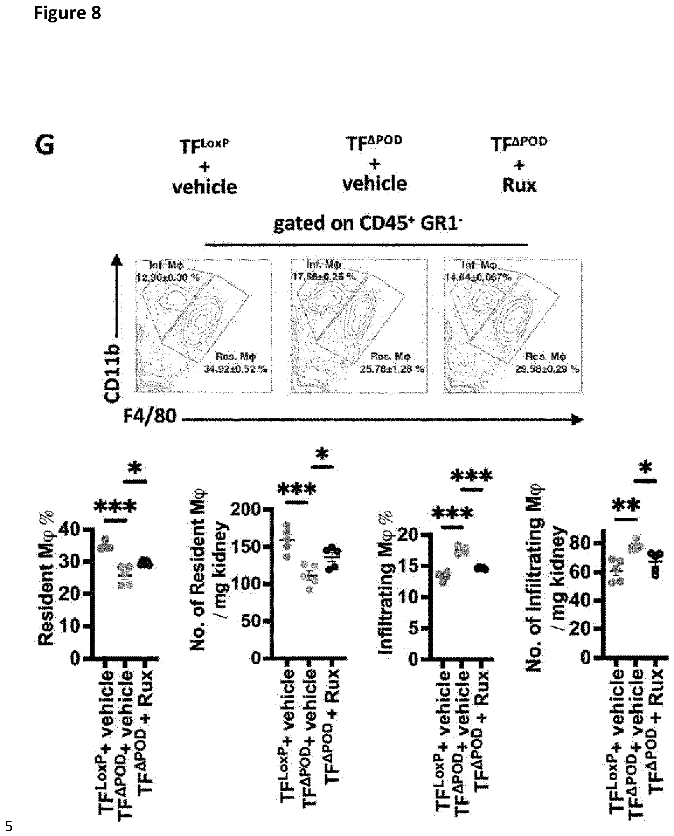

**Figure 8** **shows sterile inflammation induced upon TF loss is reversed by ruxolitinib.**
   **(A)** Representative immunoblots image of p-JAK1, JAK1, p-JAK2, JAK2, p-TYK2, TYK2, p-STAT1, STAT1, p-STAT2, STAT2, β-ACTIN in human TF-expressing (TF^{WT}) and TF-knockout (TF^{KO}) podocytes exposed to DMSO (solvent, control) or 100 nM ruxolitinib (Rux) for 24 h. For each phosphorylated protein the non-phosphorylated form is shown for normalization. β-ACTIN serves as a loading control. **(B)** Bar graph represents fold changes of ISGs mRNA level from human podocytes treated with DMSO or 100 nM Rux for 24 h. Bar graph summarizing data (mean ± SEM of 3 independent experiment; each dot represents one independent experiment). **(C)** Dot plot representing urine ACR levels obtained from 16 weeks old TF^{LoxP} and TF^{ΔPOD} mice treated with vehicle (PEG and Dextrose) or ruxolitinib (Rux; 30mg/kg/day) for 10 days. **(D)** Representative immunoblot images of p-STAT1, STAT1, p-STAT2, STAT2, β-ACTIN in whole kidney protein lysates from 16 weeks old TF^{LoxP} and TF^{ΔPOD} mice treated with vehicle or Rux. For each phosphorylated protein the non-phosphorylated form is shown for normalization. β-ACTIN serves as a loading control. **(E)** Representative images of glomerular sections with PAS-stain or immunohistochemical WT1 staining obtained from 16 weeks old TF^{LoxP} and TF^{ΔPOD} mice treated with vehicle (PEG and Dextrose) or ruxolitinib (Rux; 30mg/kg/day). Dot plot with mean ± SEM summarizing data for WT1⁺cells per glomerular cross sections (gcs; at least 10 gcs per mice were analyzed) or FMA in percent; each dot represent data from one mouse; scale bar: 20 µm. **(F)** Heatmap representing the significantly altered (**P*<0.05; one-way ANOVA) mRNA foldchange of DAMPS, PRRs and cytokines in whole kidney RNA from 16 weeks old TF^{LoxP} and TF^{ΔPOD} mice treated with vehicle or Rux. 18sRNA level were used for data normalization. Z-score represents up and down regulation of genes. **(G)** FACS immunophenotyping of infiltrating (F4/80^{low}CD11b^{hi}) and resident (F4/80^{hi}CD11b^{low}) macrophages in whole kidneys of 16 weeks old TF^{LoxP} and TF^{ΔPOD} mice treated with vehicle or Rux. Dot plot summarizing data (mean ± SEM; each dot represents data from one mouse). **B, C, E, F, G:** *P* values determined using one-way ANOVA with Tukey-*post hoc* comparison (**P*<0.05, ***P*<0.002, ****P*<0.001).

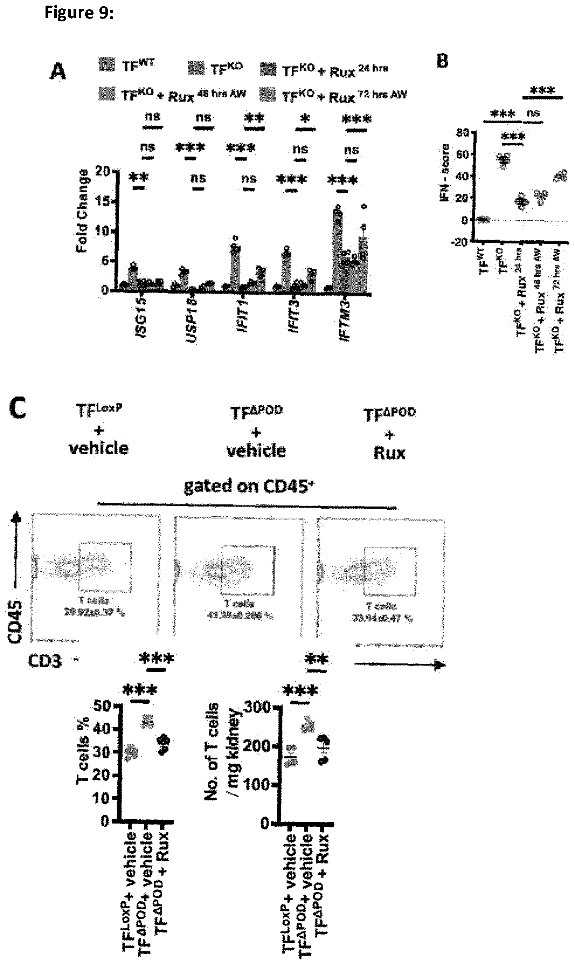

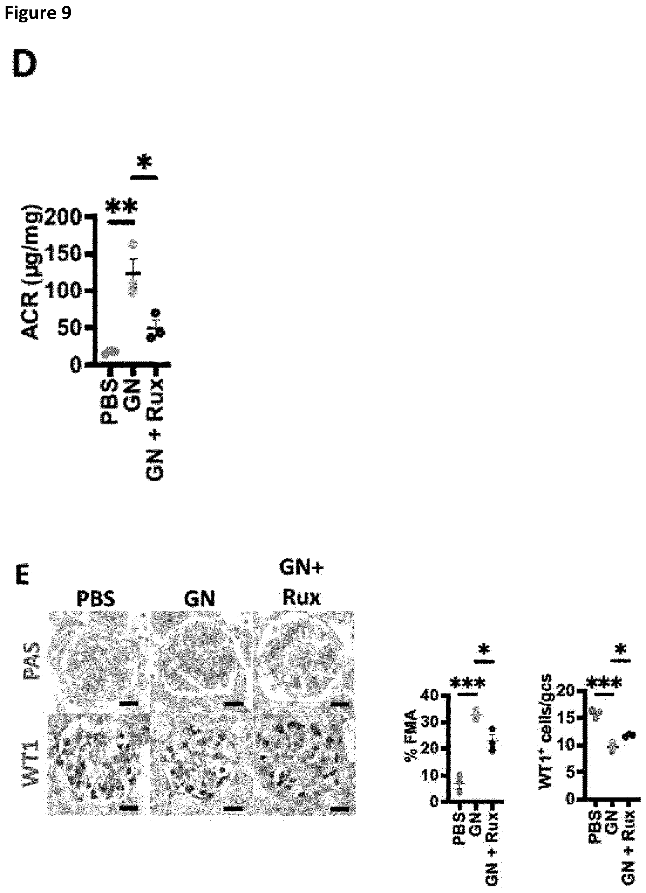

**Figure 9** **shows podocytes TF loss induced disruption of renal immune homeostasis is reversed by ruxolitinib.**
   **(A)** Bar graph represents fold changes of ISGs mRNA level in human podocytes exposed to DMSO (solvent, in TF^{wt} and TF^{KO}), to 100 nM ruxolitinib (Rux) for 24 h, or to 100 nM ruxolitinib for 24 h and follow up after 48 h or 72 h after washing ruxolitinib (AW). **(B)** Dot plot represents IFN score calculated based on the relative expression levels of ISGs shown in panel A. Bar graph and Dot plot summarizes mean ± SEM of 4 independent experiment; each dot represents one independent experiment. **(C)** FACS immunophenotyping of T cells in whole kidneys of TF^{LoxP} and TF^{ΔPOD} treated with vehicle and Rux. Dot plot summarizing data (mean ± SEM; each dot represents data from one mouse). **(D, E)** Dot plot (D) representing urine ACR (albumin-creatinine-ratio) levels and representative images of glomerular sections (D) with PAS-stain or immunohistochemical WT1 staining obtained from 10 days glomerulonephritic (GN; nephrotoxic serum injected at 8 weeks old) treated with ruxolitinib for 5 days after GN induction or control (PBS injected) wild type mice. Dot plot with mean ± SEM summarizing data for ACR (D) and WT1+cells per glomerular cross sections (gcs; at least 15 gcs per mice were analyzed) or FMA in percent (E); each dot represent data from one mouse; scale bar: 20 µm. **A-D:** *P* values determined using one-way ANOVA with Tukey-*post* hoc comparison (**P*<0.05, ***P*<0.002, ****P*<0.001).

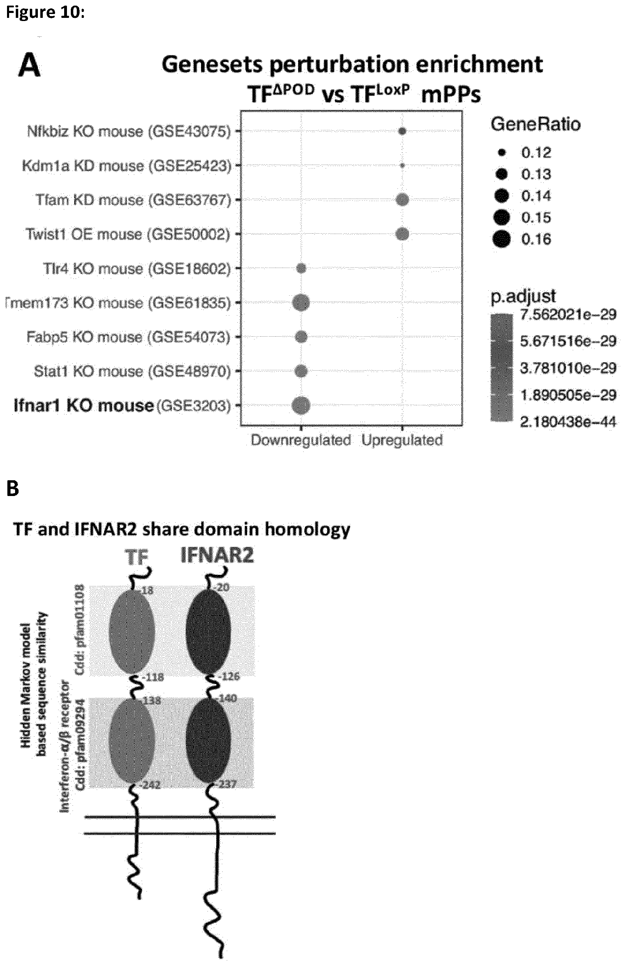

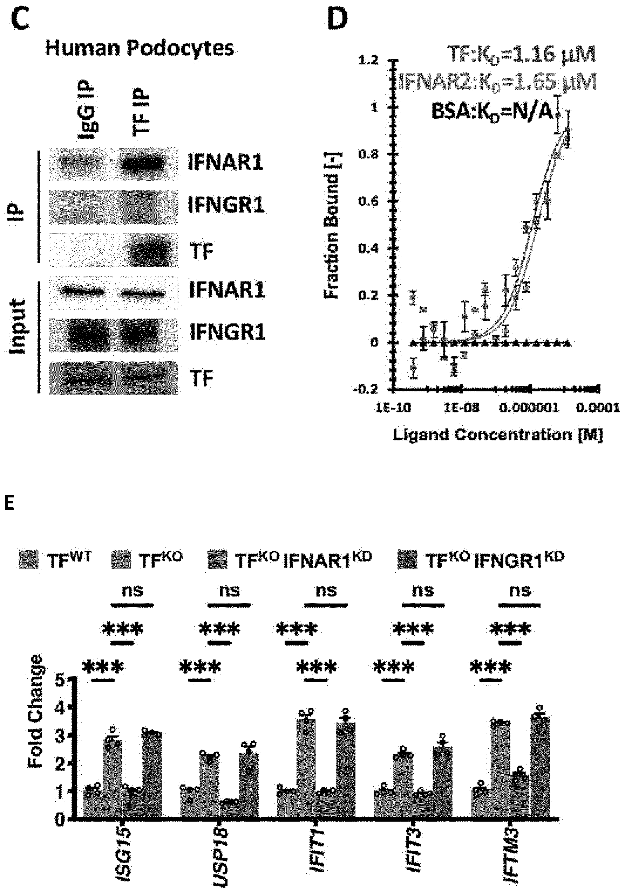

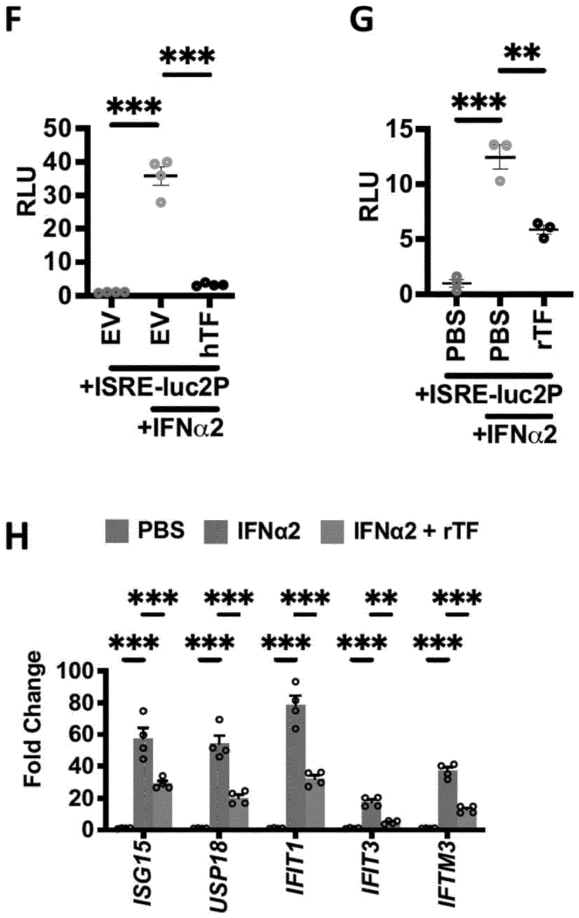

**Figure 10** **shows TF interacts with IFNAR1 and restricts the interferon response.**
   **(A)** Dot plots represent significant overlap of downregulated or upregulated DEGs from indicated knockout mice with DEGs of TF^{ΔPOD} versus TF^{LoxP} mPP based on Enrichr analyses. **(B)** Illustration depicts conserved cytokine receptor domains in tissue factor and IFNAR2. **(C)** Representative immunoblots image of IFNAR1, IFNGR1 and TF from TF coimmunoprecipitation (IP) and input of human podocytes lysates. Input serves as a loading control. **(D)** Line-graph representing fraction of TF bound to IFNAR1 (left side curve) or albumin (bottom line, no binding), and IFNAR2 bound to IFNAR1 (right side curve) by microscale thermophoresis binding assay. **(E)** Bar graph representing fold changes of ISGs mRNA in human podocytes treated with control siRNA (TF^{WT} or TF^{KO}), silFNAR1 (TF^{KO}IFNAR1^{KD}) or silFNGR1 (TF^{KO}IFNGR1^{KD}). Bar graph summarizing data (mean ± SEM of 4 independent experiment; each dot represents one independent experiment). **(F)** Dot plot summarizing relative luciferase unit (RLU) in HEK293T cells transfected with ISRE-luc2P, empty vector (EV), and human TF (hTF) and stimulated with IFNa2 (500 U/mL, 24 h). Dot plot summarizing data (mean ± SEM; each dot represents one independent experiment). **(G)** Dot plot summarizing RLU in HEK293T cells transfected with ISRE-luc2P and stimulated with IFNa2 (500 U/mL) and treated with recombinant TF (rTF; extracellular domain of TF; 100 nM) for 24 h. Dot plot summarizing data (mean ± SEM; each dot represents one independent experiment). **(H)** Bar graph summarizing fold changes of ISGs mRNA level in THP1 cells stimulated with IFNa2 (500 U/mL; control: PBS, blue) without or with rTF (100 nM) for 24 h. Mean ± SEM of three independent experiments; each dot represents one independent experiment. **E, H:** P values determined by two-way ANOVA with Tukey-*post hoc* **comparison.F, G:** P values determined by one-way ANOVA with Tukey-*post hoc* comparison (**P*<0.05, ***P*<0.002, ****P*<0.001).

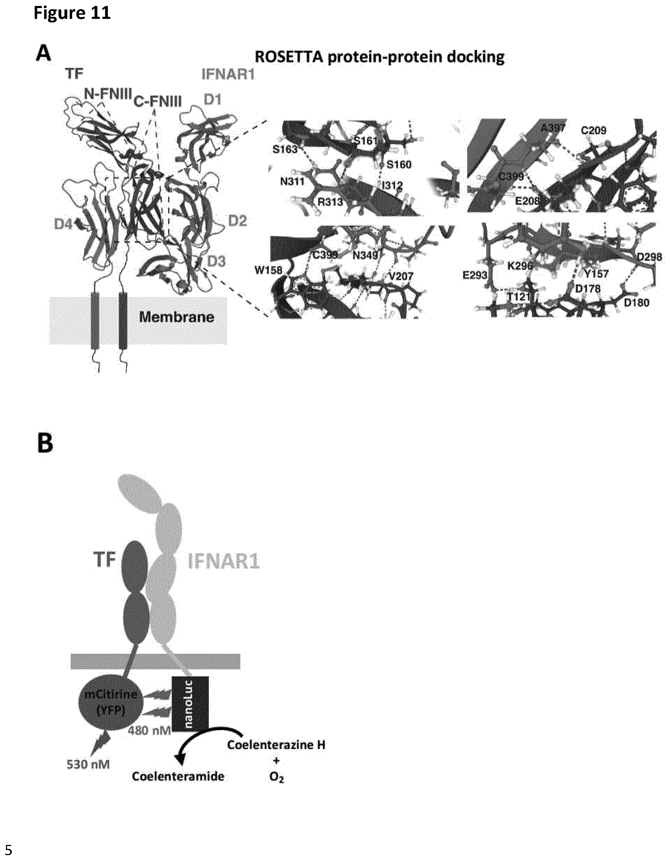

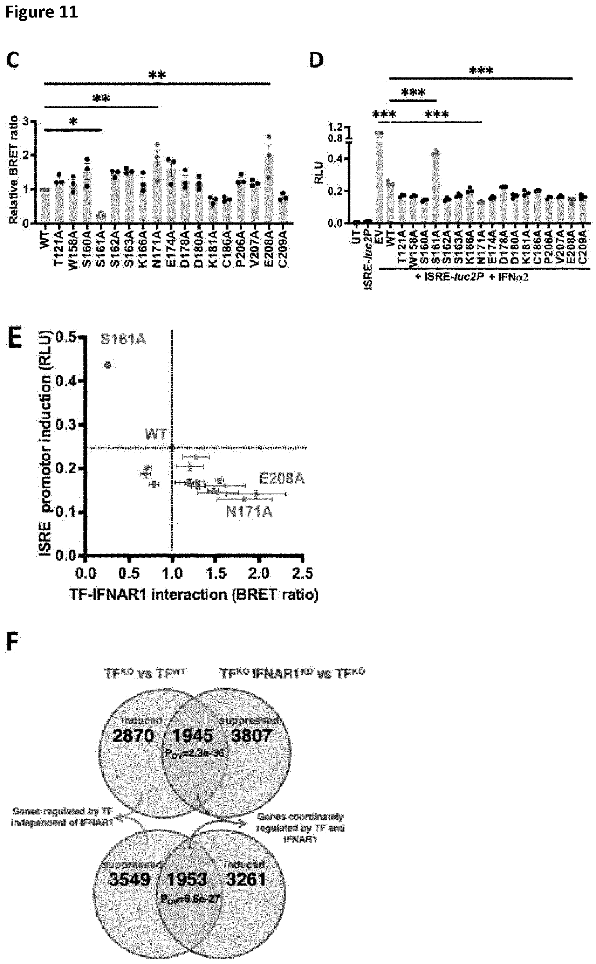

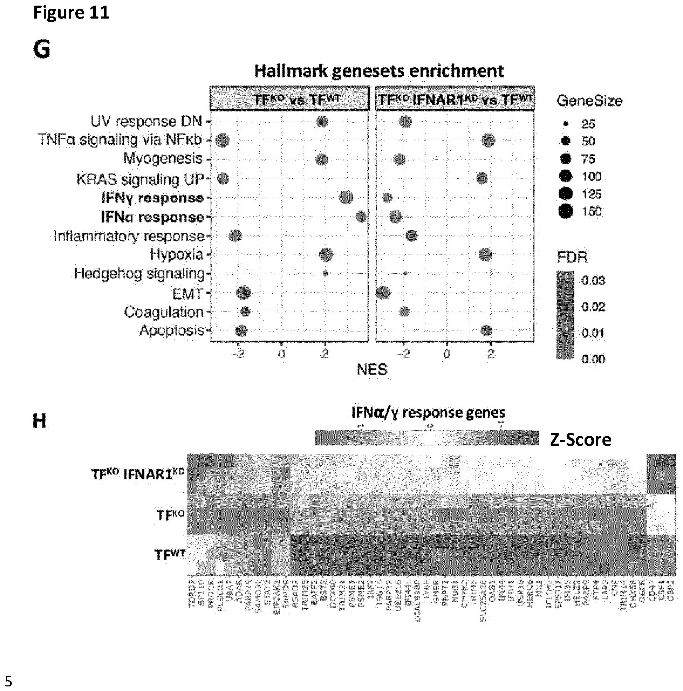

**Figure 11** **shows TF interacts with IFNAR1 via its C-FNIII domain and restricts the interferon response. (A)** Computational model of the TF-IFNAR1 complex depicting interaction of IFNAR1 domains (D1-D4) with TF domains (N-terminal fibronectin domain III - N-FNIII) and C-terminal fibronectin domain III - C-FNIII) at multiple sites. **(B)** Scheme of the bioluminescence resonance energy transfer (BRET) to detect the TF (mCitirine labelled) and IFNAR1 (nanoLuc labelled) interaction. **(C)** Dot plot represents relative BRET ratio measured by expressing IFNAR1-nanoLuc and mutant TF-mCitirine constructs in HEK293T TF knockout (TF^{KO}) cells. Dot plot summarizing data (mean ± SEM; each dot represents one independent experiment). **(D)** Dot plot represents relative luciferase units (RLU) determined in cells expressing mutant TF-mCitirine and ISRE-luc2P in HEK293T TF^{KO} cells, which were stimulated with IFNα2 (500 U/mL) for 24 h. **(E)** Dot plot representing the relation of relative BRET ratio and RLU for each TF-mutant. Dot plot summarizing data (mean ± SEM; each dot represents three independent experiment). **(F)** Venn diagram representing DEGs in TF^{KO} human podocytes compared to TF^{WT} human podocytes (left circle) and in TF^{KO} human podocytes compared TF^{KO} with concomitant IFNAR1 knockdown (TF^{KO}IFNAR1^{KD}, right circle). *P*-value indicates significant overlap (Pov, hypergeometric test). **(G)** Dot plot representing significantly enriched common hallmark gene sets from GSEA between TF^{KO} versus TF^{WT} and TF^{KO}IFNAR1^{KD} versus TF^{KO} human podocytes, represented as normalized enrichment score (NES). **(H)** Heat map representing selected DEGs related to the IFNa/y response when comparing TF^{WT}, TF^{KO}, and TF^{KO} IFNAR1^{KD} human podocytes. Z-score represents up and down regulation of genes. **C, D:** *P* values were determined using a one-way ANOVA with Tukey*-post hoc* comparison (**P*<0.05, ***P*<0.002, ****P*<0.001).

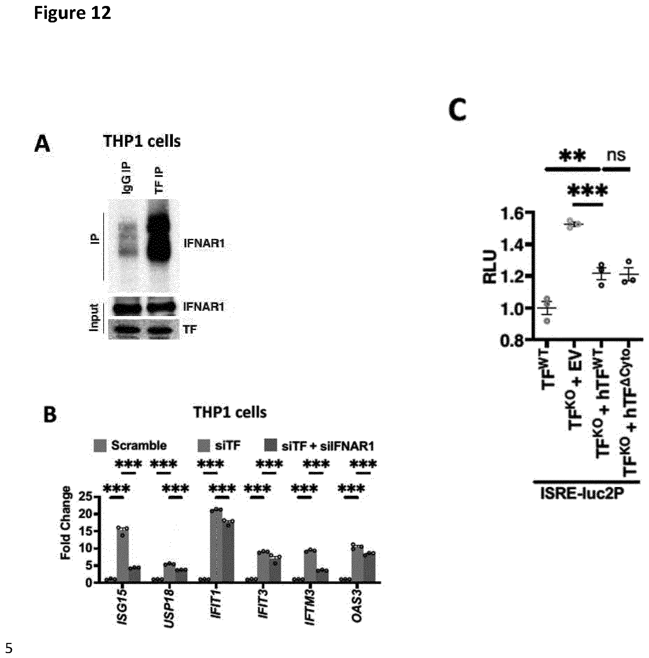

**Figure 12** **shows IFNAR1 knockdown reversed TF loss induced ISGs expression.**
   **(A)** Representative immunoblots image of IFNAR1 and TF from TF coimmunoprecipitation (IP) and input of THP1 monocytes lysate. Input serves as a loading control. **(B)** Bar graph representing fold changes of ISGs mRNA in THP1 cells treated with control siRNA, siTF, siIFNAR1. Bar graph summarizing data (mean ± SEM of 3 independent experiment; each dot represents one independent experiment). **(C)** Dot plot summarizing RLU in TF^{WT} and TF^{KO} HEK293T cells transfected with ISRE-luc2P, (wild type tissue factor) hTF^{WT} or (tissue factor lacking cytoplasmic domain) hTF^{Δcyto} constructs. Dot plot summarizing data (mean ± SEM; each dot represents one independent experiment). **B:** *P* values were determined using two-way ANOVA with Tukey-*post hoc* comparison **C:** P values were determined using one-way ANOVA with Tukey-*post hoc* comparison (***P*<0.002, ****P*<0.001).

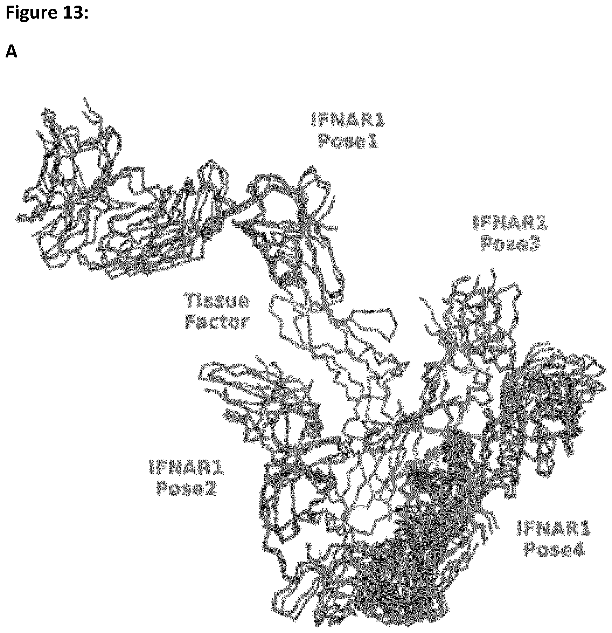

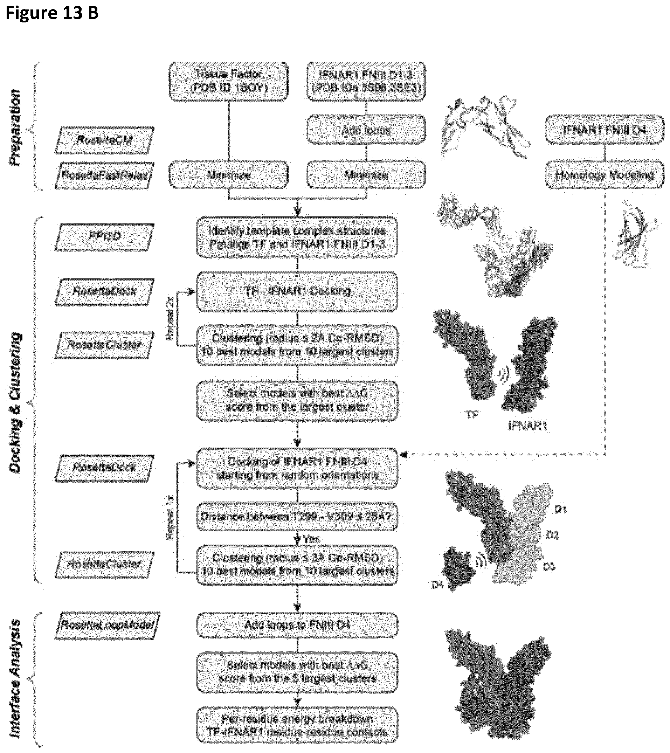

**Figure 13** **shows an overview of the computational modeling workflow for prediction of the TF-IFNAR1 complex structure.**
   **(A)** Putative binding interfaces between TF and IFNAR1 identified by searching for homologous complex structures in the protein data base (PDB) using the PPI3 webserver. The TF-IFNAR1 interaction at each binding interface was probed by high-resolution Rosetta docking and the best-scoring model was identified as most probable TF-IFNAR1 interaction model. **(B)** Overview of the computational modeling workflow for prediction of the TF-IFNAR1 complex structure. The individual steps are described in the supplemental methods.

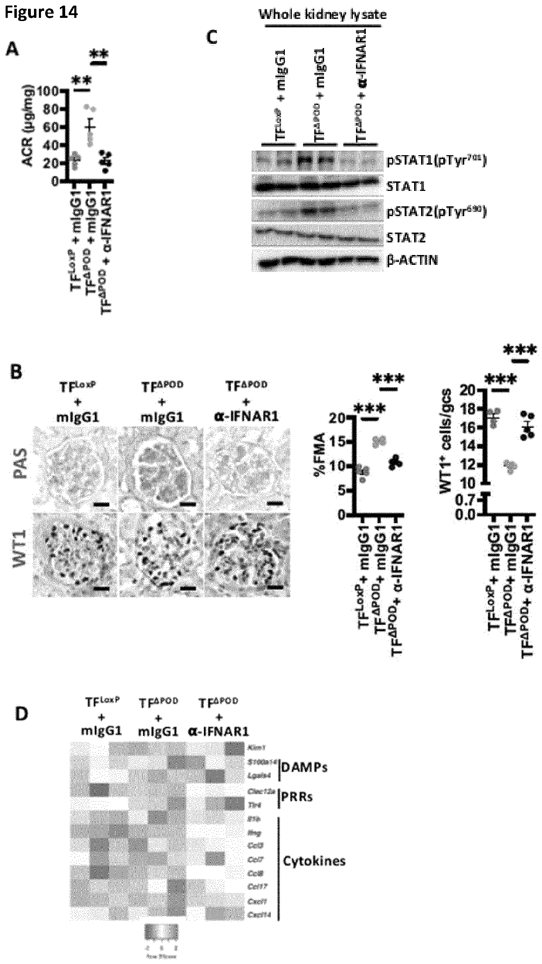

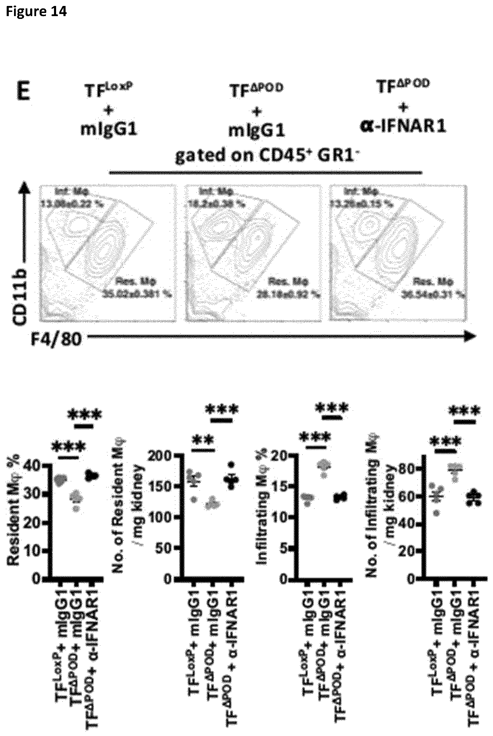

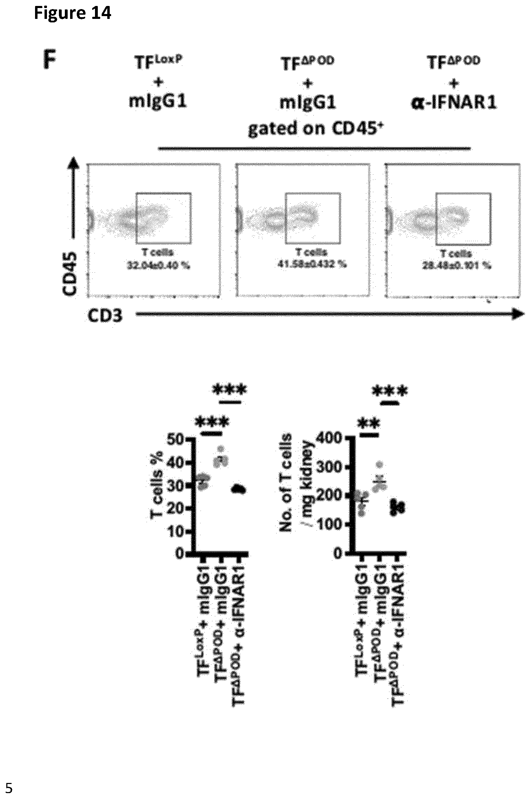

**Figure 14** **shows IFNAR1 blocking inhibited sterile inflammation induced by TF-loss in podocytes.**
   **(A,B)** Dot plot (A) representing urine ACR (albumin-creatinine-ratio) levels and representative images of glomerular sections (B) with PAS-stain or immunohistochemical WT1 staining obtained from 16 weeks old TF^{LoxP} and TF^{ΔPOD} mice treated with an anti-IFNAR1 blocking antibody (α-IFNAR1) or a control IgG1 (mlgG1) for 10 days. Dot plot with mean ± SEM summarizing data for ACR (A) and WT1⁺cells per glomerular cross sections (gcs; at least 15 gcs per mice were analyzed) or FMA in percent (B); each dot represent data from one mouse; scale bar: 20 µm. **(C)** Representative immunoblot images of p-STAT1, STAT1, p-STAT2, STAT2, β-ACTIN in whole kidney protein lysates from 16 weeks old TF^{LoxP} and TF^{ΔPOD} mice treated with α-IFNAR1 or mlgG1. For each phosphorylated protein the non-phosphorylated form is shown for normalization. β-ACTIN serves as loading control. **(D)** Heatmap representing significantly altered (**P*<0.05; one-way ANOVA) mRNA expression of selected DAMPS, PRRs and cytokines in whole kidneys of 16 weeks old TF^{LoxP} and TF^{ΔPOD} mice treated with α-IFNAR1 or mlgG1. 18sRNA level were used for data normalization.Z-score represents up and down regulation of genes. (E) FACS immunophenotyping of infiltrating (F4/80^{low}CD11b^{hi}) and resident (F4/80^{hi}CD11b^{low}) macrophages in whole kidneys of 16 weeks old TF^{LoxP} and TF^{ΔPOD} mice treated with α-IFNAR1 or mlgG1. **(F)** FACS immunophenotyping of T cells in whole kidneys of 16 weeks old TF^{LoxP} and TF^{ΔPOD} treated with α-IFNAR1 or mlgG1. Dot plot summarizing data (mean ± SEM; each dot represents data from one mouse). Dot plot summarizing data (mean ± SEM; each dot represents data from one mouse). **A, B, D, E, F:** *P* values were determined using a one-way ANOVA with Tukey-*post hoc* comparison (**P*<0.05, ***P*<0.002, ****P*<0.001).

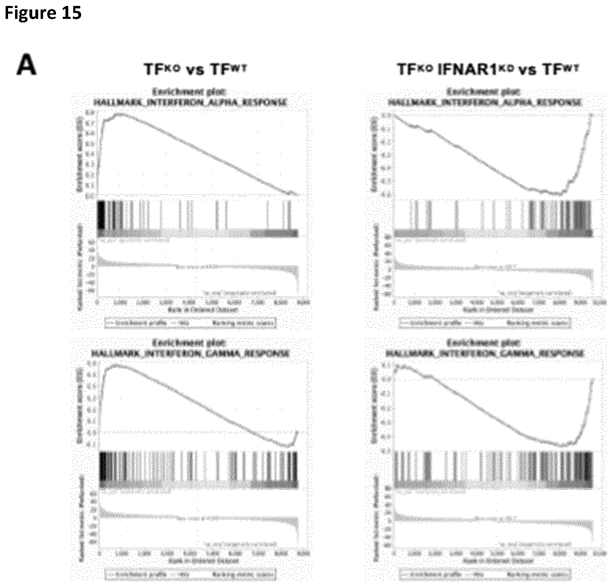

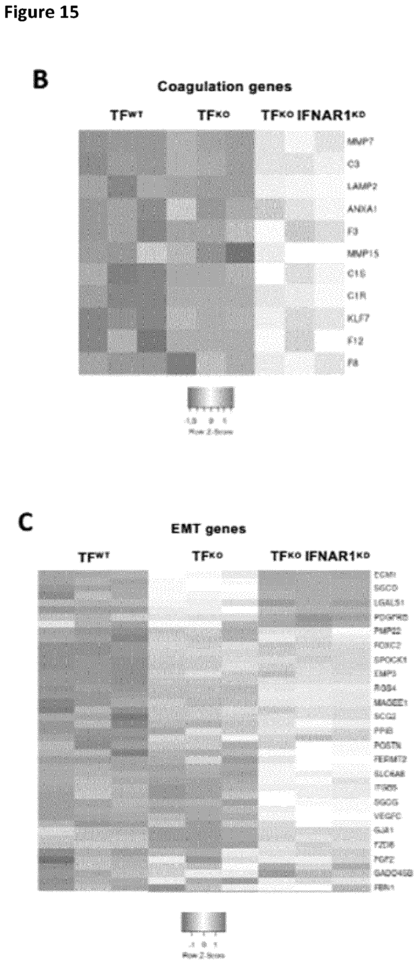

**Figure 15** **shows IFNAR1 knockdown reversed TF loss induced interferon response genes, but not coagulation and EMT genes**
   **(A)** GSEA plot representing transcriptome enrichment profiles of pre-ranked gene lists of DEGs obtained from comparison of human TF^{WT} vs TF^{KO} and TF^{KO} vs TF^{KO} IFNAR1^{KD} podocytes. **(B, C)** Heat map representing selected DEGs related to the coagulation and epithelial to mesenchymal transition pathways in TF^{WT}, TF^{KO}, and TF^{KO} IFNAR1^{KD} human podocytes.Z-score represents up and down regulated genes.

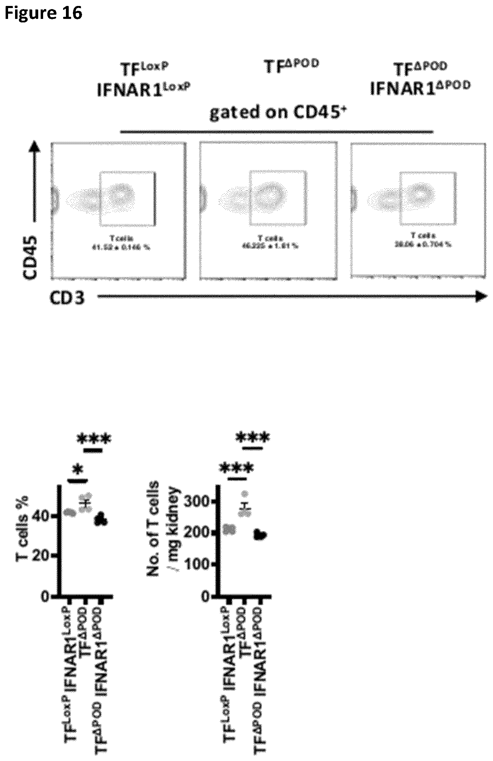

**Figure 16** **shows podocyte TF maintains renal immune homeostasis by inhibiting IFNAR1 signaling.**
   **(A)** FACS immunophenotyping of T cells in whole kidneys of TF^{LoxP}IFNAR1^{LoxP}, TF^{ΔPOD} and TF^{ΔPOD} IFNAR1^{ΔPOD} mice. Dot plot summarizing data (mean ± SEM; each dot represents data from one mouse). *P* values were determined using one-way ANOVA with Tukey-*post hoc* comparison (**P*<0.05, ***P*<0.002, ****P*<0.001).

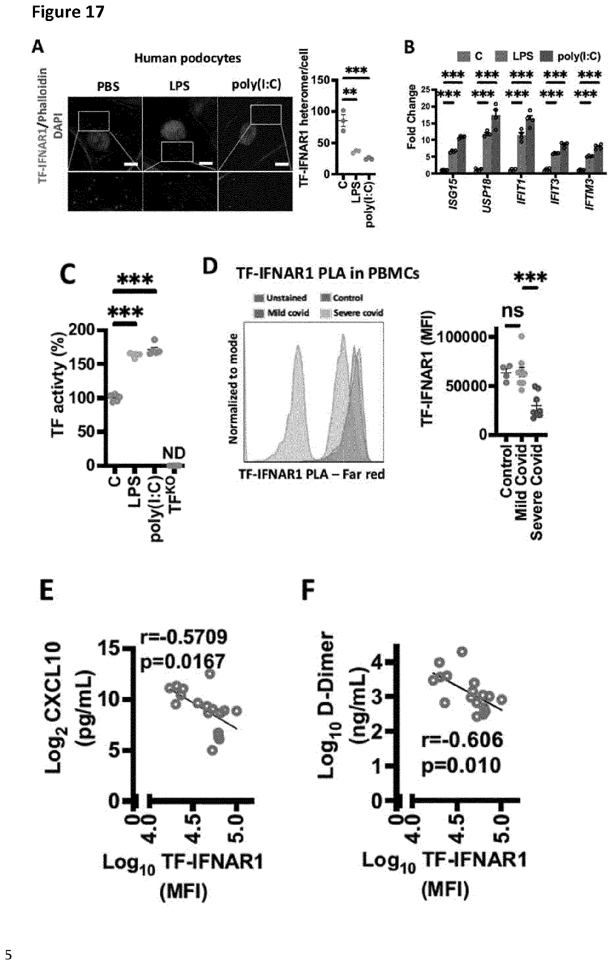

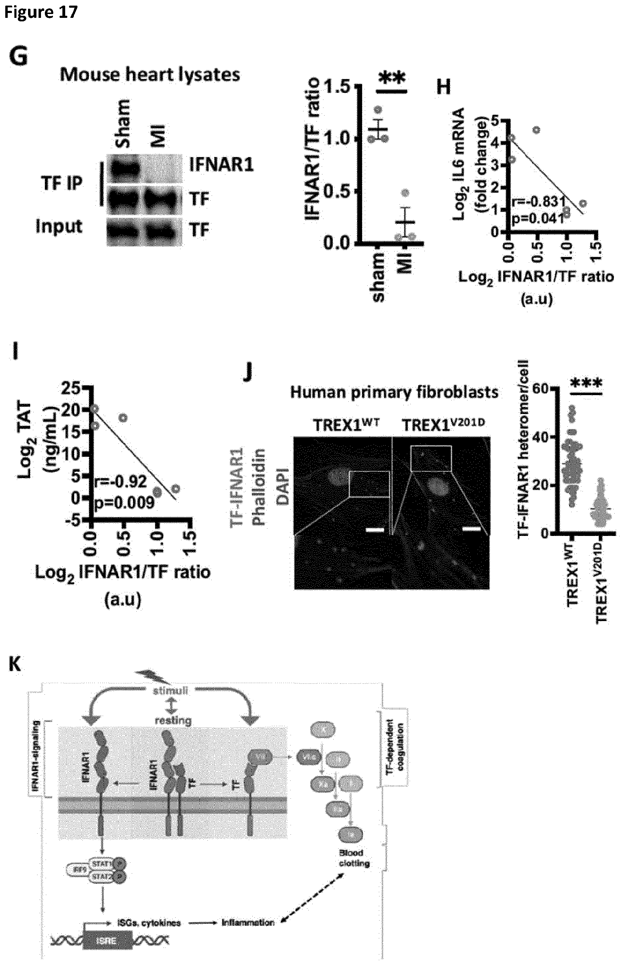

**Figure 17** **shows the TF-IFNAR1 interaction constitutes a thrombo-inflammatory switch**
   **(A)** Representative image of proximity ligation assay (PLA) in human podocytes exposed to PBS (control, C) LPS (100 ng/mL) or polylC (50 ng/mL) for 24 h. Dot plot with mean±SEM represents quantification of PLA results. Each dot represents one independent experiment. **(B)** Bar graph (mean±SEM of 3 independent experiment; each dot represents one independent experiment) summarizing data of ISGs mRNA expression in human podocytes exposed to PBS (control, C), LPS (100 ng/mL), or polylC (50 ng/mL) for 24 h. **(C)** Dot plot summarizing relative TF activity in human podocytes exposed to PBS (control, C), LPS (100 ng/mL) or polylC (50 ng/mL) for 24 h. Dot plot with mean±SEM represents quantification of TF activity. Each dot represents one independent experiment. **(D)** Representative histogram (left) of TF-IFNAR1 PLA mean fluorescence intensity (MFI, FACS) from PBMCs of unstained, control (healthy donor) and patients with mild or severe Covid-19 infection. Dot plot summarizing (with mean±SEM, each dot represents one individual) the MFI of the TF-IFNAR1 PLA signal. **(E,F)** Correlation of plasma D-dimer and CXCL10 levels with TF-IFNAR1 PLA MFI in PBMCs of controls and Covid-19 patients. **(G)** Representative immunoblot images (left of IFNAR1 and TF from TF coimmunoprecipitation (IP) and input in mouse heart lysates from control (sham operated) and acute myocardial infarction (MI) mice. Input serves as a loading control. Dot plot (right, with mean±SEM) represents the ratio of IFNAR1 to TF obtained from TF IP of mouse heart lysates. **(H, I)** Correlation analysis of heart IL6 mRNA levels and plasma TAT levels with IFNAR1/TF ratio from immunoblot of TF IP. **(J)** Immunofluorescence images (left) represents PLA signals (dots)of TF-IFNAR1 interaction with phalloidin stained cytoskeleton and DAPI (nucleus) in human primary fibroblast obtained from healthy donor (TREX1^{WT}) and a interferonopathy patient carrying TREX1 mutation (TREX1^{V201D}). Dot plot (right) summarizing PLA signals of TF-IFNAR1 interaction from two healthy donors and one interferonopathy patient. **(K)** Illustration depict TF-IFNAR1 switch that model in which TF and IFNAR1 form an inactive heteromer in resting cells, but upon cellular stimulation the TF-IFNAR1 heteromer dissociates, inducing both TF-activity and IFNAR1-dependent signaling. **A, C, D:** *P* values determined by one-way ANOVA with Tukey-*post hoc* comparison; **B:** *P* values determined by two-way ANOVA with Tukey-*post hoc* comparison (***P*<0.02, ****P*<0.001). **G, J:** *P* values determined by two-tailed Student's *t* test (**P*<0.05, ***P*<0.002, ****P*<0.001). **E, F, H, I:** Confidence interval of r (Pearson coefficient) and *P* values were calculated by linear regression; **P*<0.05. (**r*<0.05).

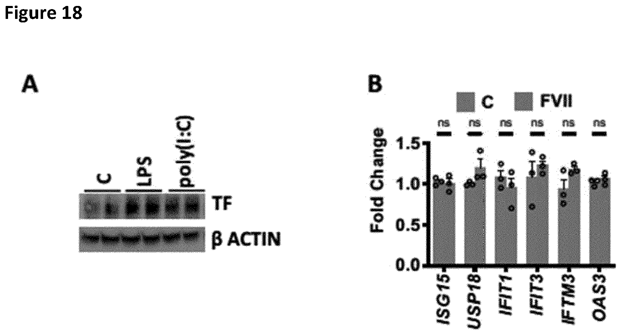

**Figure 18** **shows ISGs suppression by TF is independent of FVIIa in podocytes.**
   **(A)** Bar graph represents ISGs mRNA expression from human podocytes treated with 10 nM FVII or PBS control (C). The data shown as dot in the bar graph represent mean ± SEM of 3 independent experiments. *P* values were determined using a two-tailed Student's *t* test (ns: non-significant)

The following examples are presented to describe particular embodiments of the invention, without being limiting in scope.

### EXAMPLES

### Example 1: Loss of TF promotes renal dysfunction.

Available transcriptomic data have been analyzed to identify renal cell types expressing TF. TF expression was substantially higher in glomeruli than in the tubulointerstitium (Figure 1A) and based on single-cell RNA-sequencing (scRNA-seq) from healthy human adult kidney data, TF expression was highest in glomerular podocytes compared to other kidney resident cells (Figure 1B). Podocyte TF expression was markedly reduced in kidney biopsies obtained from patients with renal disease (Diabetic kidney disease, DKD; lupus nephritis, LN; IgA nephropathy, IgA NP; or focal segmental glomerulosclerosis, FSGS; Figure 2A) compared to those from healthy individuals. The observed reduction in podocyte TF expression was congruent with Nephroseq glomeruli transcriptomic datasets (Figure 1C to F). Likewise, glomerular TF expression was reduced in murine models of DKD (streptozotocin (STZ) induced hyperglycemia or db/db mice, analyzed after 24 weeks of hyperglycemia) and of glomerulonephritis (GN, 10 days after injection of nephrotoxic serum) (Figure 1G).

A reduction of urinary TF (uTF) in chronic kidney disease (CKD) has been previously reported (Qi et al., 1997), suggesting that loss of podocyte TF, as observed here, may result in lower uTF. Analyses of urinary samples from a subgroup of individuals with DKD or other forms of CKD (oCKD) from a large population-based cohort LIFE-Adult confirmed that uTF levels are markedly reduced in CKD (Figure 2B) and correlated positively with glomerular filtration rate (GFR), but negatively with the urine albumin creatinine ratio (ACR) (Figure 2C, D) (Loeffler et al., 2015). Likewise, reduced glomerular TF expression correlated with a reduced GFR in DKD, nephrotic syndrome, and hypertension in publicly accessible databases (Nephroseq) (Figure 1H to J). Taken together, reduced podocyte TF-expression in various glomerular diseases is associated with impaired renal function.

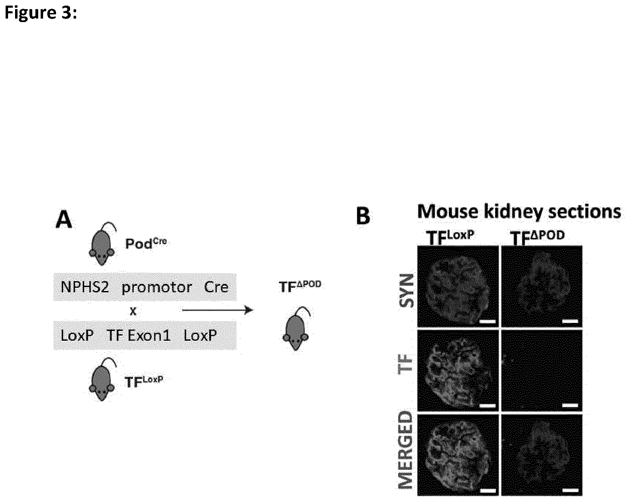

Based on these results it can be assumed that podocyte TF is required for glomerular function. To address this hypothesis, podocyte-specific TF knockout mice (TFΔPOD, Figure 3A, B) have been generated. In comparison to Cre-negative TFLoxP control mice, unchallenged TFΔPOD mice exhibited impaired renal function, as reflected by elevated albuminuria (ACR, albumincreatine ratio), increased extracellular matrix accumulation (FMA, fractional mesangial area), foot process width (FPW), and glomerular basement membrane (GBM) width, and decreased podocyte number (WT1+ cells) (Figure 2E, F). These data establish that loss of podocyte TF is sufficient to impair renal glomerular function and structure even in the absence of diseasepromoting stimuli.

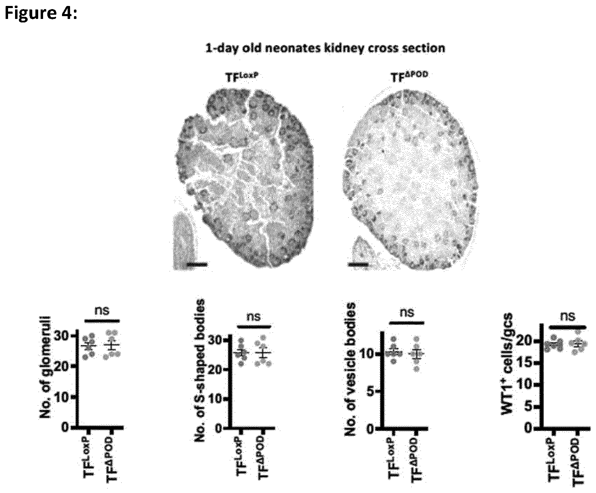

As complete TF deficiency results in embryonic lethality (Carmeliet et al., 1996), we next evaluated whether the observed glomerulopathy in TFΔPOD mice reflects impaired renal development. Glomerular development and the podocyte number were indistinguishable in 1-day-old TFΔPOD and TFLoxP neonates (Figure 4), indicating that loss of podocyte TF results in postnatal glomerulopathy.

To specifically explore the relevance of podocyte TF in renal diseases, the STZ-induced hyperglycemia model (Figure 2G) has been utilized. Upon induction of hyperglycemia, TFΔPOD mice exhibited increased albuminuria (ACR) and extracellular matrix accumulation (FMA), while podocyte number (WT1+ cells) was reduced compared to TFLoxP diabetic (DM) mice (Figure 2H, I). These findings indict that loss of TF aggravates renal injury, at least in the setting of hyperglycemia, corroborating the relevance of podocyte TF-expression.

### Example 2: Podocyte TF loss promotes interferon signaling and sterile renal inflammation.

To gain mechanistic insights into the function of podocyte TF, we performed transcriptome analysis of TFΔPOD versus TFLoxP mouse primary podocytes (mPPs, Figure 5A). Hallmark gene set enrichment by GSEA revealed positive enrichment of IFNα and IFNγ response genes, while genes related to KRAS signaling, coagulation and epithelial-to-mesenchymal transition (EMT) were negatively enriched in TFΔPOD mPPs (Figure 6A). Specifically, interferon-stimulated genes (ISGs) derived from IFNα/γ response gene sets were enriched in TFΔPOD mPPs (Figure 6B, Table S1), suggesting JAK/STAT signaling activation in these cells. Immunoblotting revealed increased p-JAK1, p-TYK2, p-STAT1 and p-STAT2 levels while p-JAK2 levels were reduced in whole kidneys obtained from TFΔPOD mice, suggesting spontaneous activation of the type I interferon (JAK1/TYK2/STAT1/STAT2) signaling pathway in the absence of podocyte TF (Figure 6C, Figure 5E). Histological analyses confirmed an enhanced glomerular interferon response, reflected by a strong induction of ISG15 and IRF9, in the absence of podocyte TF-expression (Figure 5B).

To determine whether loss of podocyte TF promotes IFN-signaling and inflammation in humans, urinary CXCL10 (a biomarker reflecting IFN-signaling) have been analyzed and systemic IL-6 in humans without and with CKD. uTF levels were negatively correlated to urinary CXCL10 and plasma IL6 (Figure 6G, H), suggesting that loss of podocyte TF (as reflected by uTF) promotes IFN-signaling and inflammation in human kidney disease, which is entirely congruent with the observations made in mice.

Next the consequences of podocyte TF deficiency in the kidneys have been determined using whole-kidney transcriptomics (Figure 5C). The biological processes enrichment from the whole-kidney transcriptome revealed that out of 34 positively enriched biological processes, 30 were related to cytokine production, immune cell chemotaxis, or inflammation, reflecting increased expression of immune response related genes in TFΔPOD kidneys compared to TFLoxP kidneys (Figure 6D, Figure 5D). Among these positively enriched biological processes danger-associated molecular patterns (DAMPs; e.g., S100a and Lgals), pattern recognition receptors (PRRs; e.g., TLR4 and Clecs) complement proteins (CPs; e.g. C1qa, C1qb and C1qc), cytokines (e.g., II1β, Cxcl1, CcI8, and CcI17), and kidney injury marker (Havcr1 a.k.a Kim1) were elevated in TFΔPOD kidneys compared to TFLoxP kidneys (Figure 6E). Whole-kidney cytokine multiplexing confirmed increased cytokine levels (IFNγ, CCL17, CCL20, CCL22, CCL3, CXCL1 CXCL10, IL1β, IL12p40, IL15, IL6, IL2, CCL5, CXCL9, IL17, and IL13) which are required for macrophage and T cell recruitment, activation, and proliferation in TFΔPOD kidneys (Figure 6F, Table S2).

We then scrutinized whether spontaneous JAK/STAT activation and associated elevated DAMPs and cytokine levels alter renal immune homeostasis in TFΔPOD kidneys. Whole-kidney immunophenotyping by FACS revealed more T cells and infiltrating macrophages (F4/80lowCD11bhi) and fewer tissue-resident macrophages (F4/80hiCD11blow) in TFΔPOD kidneys than in TFLoxP kidneys (Figure 6I, Figure 5F). This reflects an altered immune homeostasis in TFΔPOD kidneys.

**Table S1. List of differentially regulated IFN α/γ response genes in TF^{ΔPOD} mPP.**

| **Gene symbol** | **log2FoldChange** | **pvalue** |
|---|---|---|
| Oas3 | 1.658108 | 1.51E-15 |
| Rtp4 | 1.515524 | 4.60E-45 |
| Oas2 | 1.456095 | 1.88E-07 |
| Usp18 | 1.208008 | 4.80E-28 |
| Zbp1 | 1.190253 | 1.66E-08 |
| Ddx60 | 1.159151 | 1.80E-10 |
| Irf7 | 1.094069 | 1.64E-20 |
| Isg15 | 1.058094 | 4.51E-21 |
| Rsad2 | 1.037865 | 7.68E-09 |
| Ifi44 | 0.990592 | 5.77E-13 |
| Xaf1 | 0.787408 | 1.78E-13 |
| Cmpk2 | 0.725498 | 3.54E-07 |
| Dhx58 | 0.708972 | 5.85E-05 |
| Stat2 | 0.625977 | 4.13E-14 |
| Ifitm3 | 0.551432 | 7.67E-12 |
| Ifit2 | 0.543331 | 1.41E-09 |
| Stat1 | 0.538758 | 4.06E-07 |
| Lgals3bp | 0.507987 | 2.88E-14 |
| Bst2 | 0.490786 | 1.18E-09 |
| Helz2 | 0.438816 | 0.000374 |
| Rnf213 | 0.433915 | 7.96E-06 |
| Irf9 | 0.427945 | 2.91E-08 |
| Uba7 | 0.404923 | 4.32E-05 |
| Psmb8 | 0.357848 | 0.000749 |
| Ifih1 | 0.348029 | 0.000101 |
| Tnfaip2 | 0.34783 | 0.000397 |
| Herc6 | 0.34314 | 0.000109 |
| Ifi27 | 0.342622 | 9.76E-05 |
| Ifit3 | 0.33989 | 1.09E-06 |
| Gbp2 | 0.319374 | 0.001046 |
| Trim25 | 0.311934 | 0.000132 |
| Parp9 | 0.300597 | 0.000665 |
| Gmpr | 0.240309 | 0.000404 |
| Serping1 | -0.91859 | 2.48E-08 |

**Table S2. Mouse cytokine 44-plex discovery assay of whole kidney tissue homogenate.**

| **Cytokine** | **TF^{LoxP}** | | | | | **TF^{ΔPOD}** | | | | | **Student-*t* test** |
|---|---|---|---|---|---|---|---|---|---|---|---|
| **Eotaxin** | 3.38 | 3.32 | 3.01 | 3.42 | 1.07 | 6.00 | 4.39 | 5.64 | 5.14 | 4.52 | 0.00382378 |
| **CCL17** | 1.55 | 1.02 | 1.66 | 0.93 | 1.45 | 1.68 | 2.27 | 1.83 | 2.76 | 1.70 | 0.02362055 |
| **CCL20** | 7.38 | 8.95 | 8.56 | 7.96 | 7.63 | 8.41 | 11.34 | 9.85 | 10.88 | 7.91 | 0.07791491 |
| **CCL22** | 4.62 | 3.47 | 5.20 | 2.97 | 2.50 | 4.74 | 9.05 | 8.09 | 9.88 | 4.43 | 0.03231822 |
| **CCL3** | OOR< | 6.96 | 9.78 | 12.02 | OOR< | 28.18 | 27.70 | 42.58 | 19.94 | 27.70 | 0.00399174 |
| **CCL5** | 8.38 | 8.79 | 8.11 | 10.85 | 5.38 | 12.17 | 15.99 | 14.51 | 14.71 | 11.49 | 0.00198147 |
| **CXCL1** | 32.47 | 33.49 | 33.90 | 24.18 | 11.26 | 39.55 | 76.14 | 52.35 | 64.64 | 48.84 | 0.00672578 |
| **CXCL10** | 10.28 | 10.41 | 9.14 | 11.49 | 8.22 | 12.85 | 13.57 | 15.45 | 17.03 | 11.17 | 0.01169281 |
| **CXCL9** | 8.59 | 15.51 | 8.23 | 18.45 | 14.51 | 27.48 | 15.06 | 27.95 | 28.68 | 13.73 | 0.04738804 |
| **EPO** | 31.18 | 30.88 | 31.59 | 29.01 | 22.13 | 40.20 | 54.95 | 49.86 | 48.80 | 31.48 | 0.01394435 |
| **IFNβ-1** | 39.31 | 50.11 | 44.67 | 48.29 | 20.64 | 74.70 | 161.62 | 106.84 | 153.87 | 80.58 | 0.01211589 |
| **IFNγ** | 17.82 | 18.95 | 17.02 | 22.25 | 8.83 | 26.33 | 36.52 | 29.15 | 30.06 | 25.91 | 0.00271957 |
| **IL-1α** | 345.85 | 387.53 | 340.40 | 418.04 | 281.82 | 429.91 | 491.29 | 470.50 | 412.09 | 424.31 | 0.01354751 |
| **IL-1β** | 7.58 | 10.90 | 8.47 | 11.92 | 4.40 | 18.43 | 40.43 | 27.60 | 23.84 | 15.66 | 0.0162707 |
| **IL10** | 11.03 | 14.45 | 13.29 | 23.12 | 3.42 | 38.21 | 52.82 | 64.87 | 38.77 | 34.28 | 0.00216066 |
| **IL12p40** | 59.18 | 53.36 | 59.54 | 78.28 | 25.38 | 72.16 | 101.28 | 100.91 | 114.27 | 74.68 | 0.01335749 |
| **IL13** | 4.23 | 6.76 | 5.07 | 7.58 | 0.92 | 17.05 | 18.92 | 20.69 | 11.67 | 16.84 | 0.00028713 |
| **IL15** | 13.95 | 17.04 | 17.04 | 24.71 | 10.96 | 25.86 | 40.92 | 52.26 | 32.48 | 29.41 | 0.01077148 |
| **IL17** | 0.11 | 0.22 | 0.17 | 0.28 | 0.12 | 0.28 | 0.38 | 0.32 | 0.30 | 0.30 | 0.00891998 |
| **IL2** | 58.12 | 55.93 | 61.62 | 56.88 | 53.43 | 90.38 | 111.94 | 103.21 | 89.21 | 84.18 | 0.00112098 |
| **IL6** | 0.35 | 0.23 | 0.06 | 0.38 | 0.00 | 0.00 | 0.75 | 0.41 | 0.49 | 0.35 | 0.07725642 |
| **IL7** | 4.07 | 4.96 | 4.96 | 8.49 | 3.06 | 7.71 | 8.96 | 9.92 | 6.78 | 8.80 | 0.01837494 |
| **IL9** | 132.67 | 141.14 | 137.12 | 164.39 | 103.38 | 168.68 | 189.71 | 190.56 | 193.27 | 163.67 | 0.00608257 |
| **IL4** | 0.10 | 0.08 | 0.10 | 0.08 | 0.10 | 0.17 | 0.26 | 0.08 | 0.10 | 0.20 | 0.10037077 |
| **VEGF** | 20.54 | 16.03 | 18.33 | 19.01 | 12.37 | 11.12 | 21.03 | 9.27 | 9.83 | 12.11 | 0.11864315 |
| **CXCL2** | 556.69 | 737.54 | 552.65 | 607.24 | 477.82 | 710.73 | 658.84 | 638.17 | 633.10 | 651.77 | 0.17385466 |
| **Fractalkine** | 1436.2 9 | 4278.2 2 | 5574.4 9 | 2566.2 6 | 3367.2 3 | 5027.1 8 | 10587.5 9 | 2144.8 7 | 3925.1 1 | 4707.9 8 | 0.29215989 |
| **IL3** | 0.26 | 0.20 | 0.26 | 0.32 | 0.29 | 0.10 | 0.39 | 0.17 | 0.07 | 0.32 | 0.43290085 |
| **IL5** | 0.07 | OOR< | OOR< | 0.24 | OOR< | OOR< | 0.03 | 0.03 | OOR< | 0.10 | 0.43426069 |
| **CCL12** | 2.60 | 3.40 | 2.75 | 4.67 | 2.29 | 3.60 | 3.06 | 5.44 | 4.37 | 2.10 | 0.44446435 |
| **6Ckine/Exodus 2** | 1263.5 2 | 2114.4 7 | 1362.4 4 | 5158.8 6 | 534.05 | 1286.2 3 | 2096.80 | 6624.1 3 | 2381.0 2 | 1611.1 5 | 0.58912836 |
| **IL16** | 70.64 | 82.22 | 78.03 | 120.49 | 71.72 | 72.70 | 65.35 | 98.64 | 89.15 | 69.23 | 0.63175893 |
| **G-CSF** | 0.49 | 0.03 | 0.49 | 0.41 | 0.33 | 0.03 | 0.87 | 0.18 | 0.03 | 0.64 | 1 |
| **GM-CSF** | OOR< | OOR< | OOR< | OOR< | OOR< | OOR< | OOR< | OOR< | OOR< | OOR< | NA |
| **IL12p70** | OOR< | OOR< | OOR< | 1.77 | OOR< | OOR< | OOR< | OOR< | OOR< | OOR< | NA |
| **LIF** | OOR< | OOR< | OOR< | 0.12 | OOR< | OOR< | OOR< | 0.08 | OOR< | OOR< | NA |
| **LIX** | OOR | OOR | OOR | OOR | OOR | OOR | OOR | OOR | OOR | OOR | NA |
| **CCL2** | 2.63 | OOR | OOR | 2.63 | 0.36 | OOR | OOR | OOR | OOR | 1.50 | NA |
| **M-CSF** | OOR | OOR | OOR | OOR | OOR | OOR | OOR | OOR | OOR | OOR | NA |
| **CCL4** | OOR< | OOR< | OOR< | OOR< | OOR< | OOR< | OOR< | OOR< | OOR< | OOR< | NA |
| **TNFα** | OOR< | OOR< | OOR< | OOR< | OOR< | OOR< | OOR< | OOR< | OOR< | OOR< | NA |
| **IL11** | OOR< | OOR< | OOR< | OOR< | OOR< | OOR< | OOR< | OOR< | OOR< | OOR< | NA |
| **IL20** | OOR< | OOR< | OOR< | OOR< | OOR< | OOR< | OOR< | OOR< | OOR< | OOR< | NA |
| **CCL19** | OOR< | OOR< | OOR< | 13.94 | OOR< | OOR< | 8.96 | 8.96 | 12.78 | OOR< | NA |
| **TIMP1** | OOR< | OOR< | OOR< | 28.99 | OOR< | OOR< | OOR< | OOR< | OOR< | OOR< | NA |

| | | | | | | | | | | | |
|---|---|---|---|---|---|---|---|---|---|---|---|
| OOR - out of range for detection | | | | | | | | | | | |

### Example 3: TF restricts JAK/STAT-signaling dependent sterile renal inflammation.

To determine whether the spontaneous sterile inflammation in TFΔPOD kidneys depends on JAK/STAT signaling, TF knockout (TFKO) human podocytes have been treated with ruxolitinib (a JAK1/2 inhibitor), which inhibited STAT1/2 activation and reduced ISGs expression in TFKO podocytes (Figure 8A, B). The effect of ruxolitinib was transient, as upon discontinuation of ruxolitinib ISGs expression gradually increased again (Figure 9A, B).

We next determined whether pharmacological inhibition of JAK1/2 activity using ruxolitinib reduces renal STAT signaling and related changes in gene expression and renal injury in TFΔPOD mice. Ruxolitinib prevented STAT1/2 activation and reduced albuminuria, podocyte loss, glomerular damage, and the expression of Kim1, DAMPs (S100a14 and Lgals4), PRRs (Clec12a and TLR4) and cytokines in TFΔPOD mice compared to vehicle treatment (Figure 8C to F). Additionally, ruxolitinib reduced the frequencies of T cells and proinflammatory infiltrating macrophages while increasing that of tissue-resident macrophages in TFΔPOD kidneys (Figure 8G, Figure 9C).

To determine whether disease-associated TF loss and associated glomerular damage can be rescued by JAK1/2 inhibition, a murine model of glomerulonephritis (GN) has been used, as both human and murine GN is associated with reduced podocyte TF-expression (Figure 1G, Figure 2A). Five days after inducing GN, treatment with ruxolitinib was initiated for further 5 days, which reduced albuminuria (ACR) and glomerular damage, while increasing podocyte number (Figure 5D), reflecting glomerular protection. Collectively, these results show that loss of podocyte TF promotes via JAK/STAT signaling glomerulopathy and sterile kidney inflammation.

### Example 4: TF interacts with IFNAR1.

The in vitro and in vivo interventions with ruxolitinib (Figure 8) demonstrated a functional interaction between podocyte TF and the interferon response. Upon comparison of differentially expressed genes (DEGs) identified between TFΔPOD and TFLoxP mPPs (Figure 6A) to DEGs identified in other genetic mouse models using Enrichr (Kuleshov et al., 2016), a striking overlap for changes have been observed in models with an impaired interferon response (Figure 10A). In particular, the genes upregulated in TFΔPOD mPPs were significantly downregulated in mouse Ifnar1-knockout B cells. Additionally, gene expression datasets for genetically impaired ISGs regulating transcription factors, such as Stat1 (in macrophages), stimulator of interferon genes (STING or Tmem173; in bone marrow-derived dendritic cells, BMDCs) or Fabp5 (in BMDCs), were negatively correlated with DEGs in TFΔPOD mPPs (Figure 10A). These comparative analyses support a functional interaction between podocyte TF and interferon signaling, particularly IFNAR1 signaling.

These data raise the question of how TF restricts JAK/STAT activation in podocytes. TF exhibits sequence and structural homology with cytokine receptors (Bazan, 1990; Chill et al., 2003; Harlos et al., 1994; Muller et al., 1996). Specifically, TF and IFNAR2 are evolutionary related homologous proteins sharing conserved extracellular pfam domains (Renauld, 2003; Sonnhammer et al., 1998) (Figure 10B). Because IFNAR1 is known to interact with IFNAR2, it was assumed that the structural similarity between TF and IFNAR2 facilitates the interaction of TF with IFNAR1, inhibiting interferon signaling in podocytes. Indeed, immunoprecipitation, revealed an interaction of TF with IFNAR1 but not with IFNGR1 (Figure 10C). A microscale thermophoresis binding assay confirmed the direct binding of TF to IFNAR1 with a dissociation constant (KD) slightly lower than for IFNAR1-IFNAR2 binding (1.16 µM versus 1.65 µM; Figure 10D). The KD for the TF-IFNAR1 interaction (1.16 µM) is in the range of KD values for known IFNAR1-specific ligands such as IFNα1, IFNα2, IFNα8, IFNα21, IFNβ, IFNω), and IFNτ2 (KD values of 2.5, 5, 2.2, 2.5, 0.05, 0.40, and 10 µM, respectively) (Jaks et al., 2007; Lamken et al., 2004), supporting the physiological relevance of the TF-IFNAR1 interaction. These data support the binding of TF to IFNAR1, which is congruent with a model in which TF and IFNAR1 form a heteromer in podocytes, suppressing IFNAR1 signaling in these cells.

### Example 5: TF dampens ISGs by antagonizing IFNAR1.

To determine whether and to what extent the proinflammatory effect observed upon TF deletion in podocytes depends on increased IFNAR1 signaling but not on IFNGR1 signaling, IFNAR1 or IFNGR1 have been knocked down in TF^{KO} human podocytes (TF^{KO} IFNAR1^{KD} and TF^{KO} IFNGR1^{KD} podocytes). The ISGs expression induced in TF-deficient podocytes was abrogated by silFNAR1 but not by siIFNGR1, corroborating that TF specifically interferes with IFNAR1 signaling in podocytes (Figure 10E). The interaction of TF with IFNAR1 is not specific to podocytes, as we observed likewise a TF-IFNAR1 interaction in monocytes (THP1 cells; Figure 12A). As in podocytes, loss of TF increased ISGs expression, which was reduced upon concomitant IFNAR1 knock down (TF^{KD} IFNAR1^{KD}) (Figure 12B), suggesting that the TF-mediated suppression of IFNAR1 signaling has implications for immune homeostasis beyond podocytes. Taken together, these data indicate that TF binds to and dampens signaling via IFNAR1.

To determine whether increased TF levels are sufficient to dampen an IFN-triggered response, human TF and an ISRE-luc2P reporter construct have been co-expressed in HEK293T cells. Upon stimulation with IFNα2 (interferon alpha 2) for 24 h, luciferase reporter activity reflecting IFNAR1 signaling was markedly increased in the absence, but not in the presence of TF (Figure 10F). Furthermore, recombinant TF (rTF, extracellular domain of TF) dampened ISRE-promotor induction and ISGs expression by IFNα2 in HEK293T and THP1 cells, respectively (Figure 10G, H). Addition of rTF efficiently blocked the induction of ISGs-expression. Collectively, this corroborates that TF inhibits IFNα2 signaling.

### Example 6: TF C-terminal fibronectin III domain modulates IFNAR1 signaling.

To identify the molecular structures required for TF binding to IFNAR1, Rosetta protein-protein docking analysis has been performed with TF (PDB: 1BOY, comprising 2 fibronectin III (FNIII)-like domains: N-terminal FNIII (N-FNIII) and C-terminal FNIII (C-FNIII)) and IFNAR1 (PDB: 3S98, 3SE3, comprising 4 FNIII-like domains: D1, D2, D3, and a homology model of D4). This provided a putative structural model of the TF-IFNAR1 complex (Figure 7A, Figure 13A), in which 31 amino acids (AAs) of TF interact with the D1-D4 domains of IFNAR1. Of these 31 AAs, 29 were within the TF C-FNIII domain (138 to 242 AAs of TF), which exhibits structural similarity with the interferon-α/β receptor domains (pfam09294). No interaction of the N-FNIII domain of TF with IFNAR1 was observed (Table S4).

To identify crucial TF AA-residues required for the interaction with IFNAR1, alanine mutagenesis of AAs predicted to be relevant has been conducted. To measure the TF-IFNAR1 interaction, we established a bioluminescence resonance energy transfer (BRET) assay by replacing the cytoplasmic domains TF and IFNAR1 with mCitirine (YFP, acceptor) and nanoLuc (donor), respectively (Figure 11B). These reporter constructs were expressed in a HEK293T TF knockout (TF^{KO}) cell line. The S161A TF mutant largely abolished the TF-IFNAR1 interaction, while N171A and E208A TF mutants slightly increased the TF-IFNAR1 interaction (Figure 11C). Concurrently, we expressed TF-mutants along with the ISRE-luc2P reporter in HEK293T TF^{KO} cells and stimulated cells with IFNα2 to assess the functional relevance of these TF point mutations for IFNAR1 signaling. The TF S161A mutant increased IFNAR1 signaling, while the TF N171A mutant reduced IFNAR1 signaling (Figure 11D, E). These findings identify crucial AA in TF, modulating the TF-IFNAR1 interaction and IFNAR1 signaling.

### Computational modeling of the TF-IFNAR1 complex

The crystal structure of human TF (PDB ID: 1BOY) was minimized in the Rosetta ref2015 energy function and missing atoms were added using the Rosetta FastRelax protocol (Alford et al., 2017; Conway et al., 2014; Muller et al., 1996). The structure of human IFNAR1 including fibronectin (FN) III domains D1-D3 was taken from PBD IDs 3S98 and 3SE3 (Thomas et al., 2011). Missing loop regions in the IFNAR1 D1-D3 structure model were added by homology modeling with the RosettaCM protocol (Song et al., 2013). The structures of human IL-10R2 (PDB IDs: 3LQM, 5T5W), human IL-20R1 (PDB ID: 4DOH), human IL-22R1 (PDB IDs: 3DGC, 3DLQ, 6DF3), and murine IFNAR1 (PDB ID: 3WCY) served as templates in this step. In addition, a homology model of human IFNAR1 FNIII domain D4 was generated with RosettaCM using the D4 domain from the murine IFNAR1 structure (PDB ID: 3WCY) as template.

Modeling of the TF-IFNAR1 complex proceeded in two steps. In the first step, the PPI3D webserver (Dapkunas et al., 2017) was used to search for homologous protein complex structures in the PDB, which had detectable sequence similarity to TF and IFNAR1. This search identified twelve possible template structures with PDB IDs 4DOH, 6DF3, 5T5W, 6E3K, 6E3L, 1BOY, 2HFT, 1FG9, 1DAN, 2EC9, 2C4F, 3FL7 (ordered from most significant to least significant hit according to Blast e-value). Based on 3D clustering of these template structures, four possible binding interfaces between TF and IFNAR1 could be identified (shown in Figure 9A). Interface 1 involved domain D1 of both TF and IFNAR1, whereas interfaces 2 to 4 were formed between domain D2 of TF and domains D2 and D3 of IFNAR1, however, with different relative orientations of the two proteins. In the second step, each of the four binding interfaces were computationally probed by high-resolution protein-protein docking with Rosetta (Gray et al., 2003). The positions of TF and IFNAR1 in the PPI3D-generated homology models of the TF-IFNAR1 complex were perturbed by random translations and rotations of 3 Å and 8°, respectively, at the beginning of each docking run, and a total of 50,000 docking models were generated for each initial binding interface. Docking models were analyzed according to the total Rosetta energy, the Rosetta energy across the TF-IFNAR1 interface (ΔG_{separated}), the number of residues and the size of the protein surface area buried in the TF-IFNAR1 interface, as well as the shape complementarity of the TF and IFNAR1 binding surfaces. The structural model which had the best ΔG_{separated} score and the largest and most densely packed binding interface was selected as most likely model for the TF-IFNAR1 complex. Finally, to generate a model including the complete extracellular parts of TF and IFNAR1, domain D4 of IFNAR1, which is absent in the crystal structure, was docked to the best-scoring TF-IFNAR1 D1-D3 model and connected to D3 via loop modeling. This two-step docking procedure for D1-D3 and D4 domains was done because it was assumed that the D3-D4 linker is rather flexible and including all possible D3-D4 inter-domain orientations in docking would be computationally intractable. The complete modeling workflow is summarized in Figure 13B).

**Table S4. List of interacting amino acid obtained from the TF-IFNAR1 complex structure.**

| **Interacting residues of IFNAR1 (D1-D3) and TF** | | |
|---|---|---|
| TF | IFNAR1 | Type of contact |
| G120 | E293 | Vdw contact |
| T121 | E293 | sc-sc H-bond |
| T121 | E294 | bb-sc H-bond |
| K149 | W183 | Vdw contact |
| K149 | I185 | Vdw contact |
| I152 | W183 | Vdw contact |
| K166 | D298 | Vdw contact |
| K169 | W183 | Vdw contact |
| T170 | N155 | sc-sc H-bond |
| N171 | L180 | Vdw contact |
| N171 | L179 | bb-sc H-bond |
| N171 | L180 | Vdw contact |
| N171 | I185 | Vdw contact |
| T172 | E154 | Vdw contact |
| E174 | R152 | SB |
| L176 | I153 | Vdw contact |
| L176 | E154 | Vdw contact |
| L176 | I156 | Vdw contact |
| D178 | Y157 | sc-bb H-bond |
| D178 | K240 | SB |
| V179 | I295 | Vdw contact |
| V179 | K296 | bb-sc H-bond |
| D180 | D298 | sc-bb H-bond |
| D180 | I295 | Vdw contact |
| K181 | E209 | SB |
| K181 | E206 | bb-sc H-bond |
| K181 | V210 | bb-sc H-bond |
| K181 | E293 | SB |
| K181 | I295 | Vdw contact |
| V192 | W183 | Vdw contact |
| S195 | W183 | Vdw contact |

| **Interacting residues of IFNAR1 D4 (homology model) and TF (C-FNIII)** | | |
|---|---|---|
| **TF** | **IFNAR1** | **Type of contact** |
| Q110 | T350 | sc-bb H-bond |
| Q110 | N352 | sc-sc H-bond |
| W158 | V309 | Vdw contact |
| W158 | F310 | Vdw contact |
| W158 | A397 | sc-bb H-bond/Vdw contact |
| W158 | V398 | Vdw contact |
| W158 | C399 | bc-sc H-bond |
| S160 | N311 | Vdw contact/H-bond |
| S160 | I312 | bb-bb H-bond/Vdw contact |
| S160 | V398 | Vdw contact |
| S160 | C399 | bb-bb H-bond |
| S160 | E400 | sc-sc H-bond |
| S161 | V309 | Vdw contact |
| S161 | F310 | bb-bb H-bond/Vdw contact |
| S161 | N311 | bb-sc H-bond/Vdw contact |
| S161 | I312 | bb-bb H-bond/Vdw contact |
| S161 | R313 | sc-sc H-bond |
| S161 | V398 | Vdw contact |
| S162 | F310 | Vdw contact |
| S162 | D396 | Vdw contact |
| S162 | V398 | Vdw contact |
| S163 | F310 | Vdw contact |
| S163 | N311 | bb-sc H-bond |
| S163 | S395 | Vdw contact |
| S163 | D396 | bb-sc H-bond |
| S163 | V398 | Vdw contact |
| G164 | V309 | Vdw contact |
| C186 | V398 | Vdw contact |
| C186 | C399 | Vdw contact |
| P206 | N349 | Vdw contact |
| P206 | D396 | Vdw contact |
| V207 | F310 | Vdw contact |
| V207 | N349 | bb-sc H-bond/bb-bb H-bond |
| V207 | T350 | Vdw contact |
| V207 | V374 | Vdw contact |
| V207 | C376 | Vdw contact |
| V207 | D396 | bb-bb H-bond |
| V207 | A397 | bb-bb H-bond |
| V207 | V398 | Vdw contact |
| V207 | C399 | Vdw contact |
| E208 | V374 | Vdw contact |
| E208 | A397 | Vdw contact |
| E208 | C399 | Vdw contact |
| C209 | V374 | Vdw contact |
| C209 | A397 | Vdw contact |
| C209 | V398 | Vdw contact |
| C209 | E400 | Vdw contact |
| C209 | K401 | Vdw contact |
| E213 | K401 | bb-sc H-bond |
| E213 | K403 | bb-sc H-bond/sc-sc H-bond |

| | | |
|---|---|---|
| Abbreviations: sc - side chain; bb - back bone; SB - salt bridge; vdw - Vander walls interaction. | | |

### Example 7: α-IFNAR1 prevents podocyte TF loss induced sterile renal inflammation.

Considering the direct interaction of TF with IFNAR1 and inhibition of its associated signaling, it was tested whether blocking IFNAR1 *in vivo* reverses the consequences of podocyte TF deficiency. Injection of an anti-IFNAR1 blocking antibody (α-IFNAR1) for 10 days, but not injection of a nonspecific control isotype mlgG1 antibody, reduced renal STAT1/2 activation, indicating that the blocking antibody was sufficient to dampen the increased IFNAR1 signaling in TF^{ΔPOD} kidneys. The normalization of STAT1/2 activation in α-IFNAR1-treated mice was associated with marked reductions in albuminuria, podocyte loss, and glomerular damage compared to the levels in mlgG1-treated TF^{ΔPOD} mice (Figure 14A to C). Additionally, α-IFNAR1 prevented the induction of DAMPs, PRRs, and cytokines and reduced renal T-cell and infiltrating macrophage accumulation while increasing the frequency of tissue-resident macrophages in TF^{ΔPOD} kidneys compared to the IgG1 control (Figure 14D and F). Thus, inhibiting IFNAR1 reverses renal functional and inflammatory changes associated with the loss of TF expression in podocytes.

### Example 8: Concomitant loss of podocyte TF and IFNAR1 prevents sterile renal inflammation.

If altered gene expression in the absence of TF depends on IFNAR1 signaling, concomitant deletion of TF and IFNAR1 should largely reverse the effects observed in TF-deficient podocytes. Indeed, of 10317 genes differentially regulated in TF^{KO} versus TF^{WT} podocytes, 37.7% were normalized by concomitant IFNAR1 knockdown (Figure 11F). Hallmark gene sets enrichment by GSEA revealed that positively enriched gene sets, such as IFNα/γ response, UV response, myogenesis, and Hedgehog signaling, and negatively enriched gene sets, such as TNFα signaling, KRAS signaling, and apoptosis induced by TF deficiency, were normalized upon IFNAR1 knockdown (TF^{KO} IFNAR1^{KD} podocytes). Other responses induced by TF deficiency, such as coagulation, EMT, and hypoxia response, were not reversed upon concomitant IFNAR1 knockdown (Figure 11G, Figure 15A to C), suggesting that these gene sets are specifically regulated by TF independent of IFNAR1. Thus, while TF and IFNAR1 regulate some genes independently, a significant number of genes, including genes related to the IFNα/γ response, are concomitantly regulated by both receptors (Figure 11H, Table S3).

Next, to determine whether increased IFNAR1 signaling in TF-deficient podocytes causes the observed renal pathologies we generated mice lacking both TF and IFNAR1 in podocytes (TF^{ΔPOD}IFNAR1^{ΔPOD} mice). STAT1/2 activation was reduced in kidneys of TF^{ΔPOD}IFNAR1^{ΔPOD} mice, which was associated with reversal of albuminuria, podocyte loss, and glomerular damage to levels observed in wild-type control mice (TF^{LoxP}IFNAR1^{LoxP}, Figure 7A-C). Additionally, the frequency of infiltrating macrophage and of tissue-resident macrophages in kidneys of TF^{ΔPOD}IFNAR1^{ΔPOD} mice was comparable to that in wild-type control mice (TF^{LoxP}IFNAR1^{LoxP}, Figure 7D, Figure 16A). *Ex vivo* analyses of ISGs-expression in mPPs likewise confirmed reversal of ISGs induced by TF loss (TF^{ΔPOD} mPPs) upon concomitant loss of IFNAR1 (TF^{ΔPOD}IFNAR1^{ΔPOD} mPPs, Figure 7E). Thus, genetic inactivation of IFNAR1 specifically in podocytes is sufficient to reverse increased IFNAR1 signaling observed in TF^{ΔPOD} kidneys.

**Table S3. List of differentially expressed IFNα/γ response genes from human podocytes.**

| **Gene Symbol** | **TF^{WT}** vs **TF^{KO}** | | **TF^{KO} vs TF^{KO} IFNAR1^{KD}** | |
|---|---|---|---|---|
| | **log2FoldChange** | **pvalue** | **log2FoldChang e** | **pvalue** |
| ADAR | 0.488104363 | 9.66E-55 | -0.47596 | 3.53E-41 |
| ARID5B | 0.120960316 | 0.001289 | -1.15239 | 7.31E-132 |
| AUTS2 | 0.537944611 | 3.83E-05 | -0.49969 | 9.61E-05 |
| B2M | 1.021064371 | 0 | 0.168837 | 4.54E-11 |
| BANK1 | -0.497180006 | 3.78E-05 | -0.54361 | 0.000201285 |
| BATF2 | 1.926963047 | 1.09E-32 | -0.50853 | 5.19E-05 |
| BST2 | 3.833433739 | 0 | -0.88323 | 2.25E-55 |
| BTG1 | 0.146683607 | 0.000685 | 0.836053 | 4.36E-82 |
| C1R | 0.615769161 | 1.29E-37 | 0.307673 | 2.86E-12 |
| C1S | 0.594054258 | 4.18E-22 | 0.344803 | 8.41E-11 |
| CASP3 | -0.292413574 | 5.54E-14 | 0.107669 | 0.01013516 |
| CASP4 | -0.594602605 | 1.34E-27 | 0.625348 | 3.89E-32 |
| CASP7 | 0.31469395 | 2.60E-08 | -0.2015 | 0.000598105 |
| CCL2 | -3.010757954 | 1.87E-79 | -2.50319 | 5.37E-59 |
| CD47 | -0.483270598 | 5.92E-53 | -0.27009 | 1.89E-13 |
| CD74 | 0.139430203 | 3.24E-06 | -0.43375 | 2.43E-34 |
| CDKN1A | 0.385504311 | 6.58E-14 | 0.341676 | 0.003796885 |
| CFH | -0.79255447 | 4.14E-80 | -1.47938 | 1.02E-143 |
| CMPK2 | 1.932422376 | 7.64E-60 | -0.80659 | 2.44E-07 |
| CNP | 0.478751085 | 2.26E-70 | -0.34474 | 3.48E-29 |
| CSF1 | -0.524859896 | 5.55E-43 | -0.43406 | 1.82E-16 |
| CSF2RB | 4.345365343 | 2.84E-09 | -0.83297 | 0.015349991 |
| DDX58 | 1.991825213 | 8.25E-75 | -1.14381 | 3.83E-26 |
| DDX60 | 2.123653943 | 0 | -0.40947 | 7.67E-17 |
| DHX58 | 1.616097327 | 4.84E-33 | -0.91793 | 2.76E-14 |
| EIF2AK2 | 1.050832854 | 7.67E-127 | -1.15551 | 4.02E-121 |
| EIF4E3 | -0.558839461 | 3.98E-13 | 0.341125 | 1.15E-05 |
| EPSTI1 | 2.662454914 | 5.14E-80 | -1.30929 | 1.69E-31 |
| FPR1 | 1.251001662 | 1.07E-43 | -0.53818 | 5.74E-11 |
| GBP2 | -1.149348872 | 1.31E-53 | -1.05013 | 2.17E-22 |
| GBP4 | 0.608878861 | 0.004094 | -0.95303 | 8.88E-06 |
| GMPR | 1.007421806 | 7.16E-24 | -0.34113 | 8.56E-05 |
| HELZ2 | 1.971191012 | 2.82E-87 | -1.17331 | 8.56E-29 |
| HERC6 | 2.174864128 | 1.80E-120 | -0.94987 | 1.85E-74 |
| HLA-A | 0.913676469 | 2.32E-290 | 0.324391 | 1.95E-23 |
| HLA-C | 1.074970402 | 0 | 0.272947 | 2.94E-16 |
| HLA-G | 1.014954093 | 1.42E-90 | 0.316898 | 4.10E-12 |
| ICAM1 | -1.016639119 | 1.36E-86 | -0.26423 | 1.01E-05 |
| IFI35 | 1.440487972 | 9.69E-141 | -0.72109 | 4.01E-39 |
| IFI44 | 2.305965488 | 1.21E-251 | -1.05313 | 6.74E-62 |
| IFI44L | 3.914520068 | 4.92E-136 | -1.18612 | 8.40E-18 |
| IFIH1 | 1.930397749 | 1.99E-75 | -0.8377 | 3.10E-18 |
| IFITM2 | 1.501638592 | 9.29E-185 | -0.72095 | 1.46E-50 |
| IL4R | -0.481893355 | 2.10E-27 | 0.218799 | 1.58E-05 |
| IL6 | -2.258011696 | 7.58E-101 | -0.2098 | 0.000283681 |
| IRF7 | 2.130256231 | 5.04E-117 | -0.58667 | 2.32E-11 |
| ISG15 | 3.073279944 | 4.51E-249 | -0.74772 | 4.10E-17 |
| LAMP3 | 1.048982251 | 5.35E-22 | 0.248198 | 0.004651154 |
| LAP3 | 1.033785987 | 2.58E-91 | -0.68098 | 1.02E-40 |
| LGALS3BP | 0.596569961 | 3.64E-108 | -0.19932 | 6.68E-10 |
| LY6E | 1.223521467 | 1.68E-267 | -0.40931 | 1.75E-27 |
| METTL7B | 1.24582207 | 1.62E-14 | 0.357433 | 0.003910111 |
| MT2A | -0.200766529 | 4.27E-11 | 0.685869 | 1.30E-86 |
| MTHFD2 | 0.172334859 | 2.06E-05 | 0.970198 | 3.93E-135 |
| MVB12A | 0.111775815 | 0.016163 | 0.208118 | 2.06E-05 |
| MX1 | 3.172624221 | 1.29E-77 | -1.39234 | 5.96E-25 |
| MX2 | 3.878836918 | 1.73E-92 | -1.82848 | 2.04E-26 |
| MYD88 | 0.45179994 | 1.44E-18 | -0.12992 | 0.012769681 |
| NAMPT | -0.77885914 | 1.98E-157 | 0.383479 | 6.52E-33 |
| NCOA7 | 0.303512825 | 8.33E-08 | 0.493726 | 2.61E-20 |
| NFKB1 | -0.606456393 | 1.17E-55 | -0.57817 | 1.27E-32 |
| NFKBIA | -1.38707289 | 4.04E-226 | -0.79863 | 7.19E-67 |
| NLRC5 | 1.296311903 | 5.33E-81 | -0.57998 | 1.04E-19 |
| NUB1 | 0.423831588 | 8.92E-24 | -0.17201 | 0.000320143 |
| NUP93 | 0.145887085 | 1.01E-05 | 0.138216 | 9.32E-05 |
| OAS1 | 2.677344178 | 3.30E-70 | -1.33245 | 2.97E-26 |
| OASL | 3.243766982 | 5.03E-194 | 0.763248 | 7.12E-12 |
| OGFR | 0.674237997 | 1.03E-65 | -0.35407 | 3.54E-17 |
| PARP12 | 1.334347699 | 9.90E-63 | -0.39512 | 5.91E-14 |
| PARP14 | 1.126792869 | 3.60E-55 | -0.99746 | 6.72E-90 |
| PARP9 | 1.970469906 | 1.14E-108 | -1.18698 | 3.15E-37 |
| PELI1 | -0.831036712 | 2.88E-22 | 0.341236 | 0.000223101 |
| PFKP | 0.696340912 | 2.84E-196 | -0.39265 | 8.54E-33 |
| PLSCR1 | 0.669172913 | 6.34E-11 | -0.80947 | 2.78E-53 |
| PML | 0.357290547 | 2.45E-31 | -0.58312 | 4.01E-41 |
| PNPT1 | 0.577083391 | 2.27E-22 | -0.30111 | 5.97E-07 |
| PROCR | 0.354561556 | 1.96E-16 | -0.43246 | 5.74E-18 |
| PSMB10 | 0.264384648 | 0.00014 | 0.328847 | 5.85E-07 |
| PSMB8 | 0.515291238 | 4.90E-52 | 0.299413 | 3.10E-17 |
| PSME1 | 0.44280337 | 1.42E-60 | -0.14857 | 1.64E-06 |
| PSME2 | 0.662297285 | 4.90E-90 | -0.20634 | 5.71E-09 |
| PTGS2 | -1.668668506 | 4.02E-52 | 1.14291 | 3.64E-22 |
| PTPN1 | 0.320471094 | 4.01E-29 | -0.10625 | 0.000944046 |
| PTPN6 | -0.283566346 | 5.10E-05 | -0.61757 | 2.01E-14 |
| RBCK1 | 0.353600246 | 2.40E-25 | 0.701156 | 2.45E-80 |
| RIPK1 | 0.08171603 | 0.02145 | -0.09435 | 0.015102434 |
| RNF213 | 1.285618534 | 5.28E-230 | -0.83459 | 6.74E-60 |
| RSAD2 | 2.661808339 | 2.40E-77 | -0.28878 | 0.004835864 |
| RTP4 | 1.749436473 | 5.05E-25 | -1.09274 | 5.38E-13 |
| SAM D9 | 0.980418881 | 1.44E-19 | -1.03774 | 4.52E-20 |
| SAMD9L | 1.645361714 | 2.97E-59 | -1.30168 | 4.86E-90 |
| SAMHD1 | 1.542129149 | 2.24E-257 | -0.81065 | 3.79E-74 |
| SECTM1 | 2.117156164 | 1.51E-40 | -0.51069 | 1.53E-05 |
| SERPING1 | -0.588213974 | 8.93E-18 | -0.89086 | 1.64E-23 |
| SLAMF7 | -3.684559911 | 4.19E-25 | 1.162539 | 0.021995707 |
| SLC25A28 | 0.464938252 | 4.42E-17 | -0.22872 | 3.69E-05 |
| SP110 | 0.77705603 | 0.000145 | -1.09927 | 3.49E-07 |
| ST3GAL5 | 0.409406932 | 0.00023 | -0.8975 | 1.42E-13 |
| STAT1 | 0.433814228 | 4.50E-12 | -1.05318 | 3.58E-150 |
| STAT2 | 0.921909448 | 3.29E-93 | -0.69953 | 1.27E-50 |
| STAT4 | -0.403356674 | 2.98E-05 | -0.61132 | 5.19E-08 |
| TDRD7 | 0.353356839 | 1.07E-15 | -0.62903 | 8.76E-38 |
| TNFAIP2 | -0.792666678 | 4.10E-116 | -0.83623 | 1.94E-95 |
| TNFSF10 | -1.149034535 | 3.55E-39 | -2.01748 | 0 |
| TOR1B | -0.200780112 | 2.45E-06 | 0.281571 | 2.17E-11 |
| TRIM14 | 1.713888481 | 8.16E-120 | -1.2129 | 1.53E-125 |
| TRIM21 | 1.094377228 | 4.01E-79 | -0.30973 | 8.51E-08 |
| TRIM25 | 0.571472375 | 9.41E-42 | -0.11729 | 0.006534565 |
| TRIM5 | 1.266620755 | 2.42E-143 | -0.5792 | 2.28E-34 |
| UBA7 | 0.581349387 | 3.43E-25 | -0.62383 | 1.31E-22 |
| UBE2L6 | 0.962497263 | 6.47E-106 | -0.28623 | 7.12E-11 |
| UPP1 | -0.20569159 | 7.76E-05 | 2.376713 | 0 |
| USP18 | 2.776287425 | 1.55E-99 | -1.15379 | 2.91E-24 |
| VAMP5 | -1.79007783 | 1.89E-101 | -0.47517 | 6.77E-05 |
| VCAM1 | -0.618622169 | 2.19E-52 | -1.00316 | 6.07E-80 |
| XAF1 | 2.691272791 | 1.07E-103 | -1.75059 | 6.83E-57 |
| ZNFX1 | 1.053611865 | 9.45E-210 | -0.2265 | 3.36E-09 |

### Example 9: Loss of TF-IFNAR1 heteromer promotes thrombo-inflammation.

While TF has been previously linked to proinflammatory effects, the current data demonstrate an anti-inflammatory function of TF. Considering that loss of podocyte TF-expression induces spontaneous IFN-signaling, inventors assumed that the TF-IFNAR1 interaction on resting cells restricts IFN-signaling, but that upon disease stimuli the TF-IFNAR1 interaction is lost, resulting simultaneously in TF and interferon receptor (IFNR) signaling. To address this hypothesis, human podocytes have been stimulated with LPS or polylC, known triggers of IFNAR1 signaling and TF activity. Stimulation with LPS or polylC dissociated the TF-IFNAR1 heteromer (Figure 17A) and simultaneously induced ISG-expression and TF-dependent coagulation activation (Figure 17B, C). Intriguingly, stimulation of cells with fVlla had no effect on the IFN-response (Figure 18A, B). These data support a model in which TF and IFNAR1 form a heteromer on resting cells, preventing both TF and IFNAR1-dependent signaling. This heteromer is lost upon inflammatory stimulation, promoting a thrombo-inflammatory response through TF and IFNAR1-dependent signaling.

It was next scrutinized whether the TF-IFNAR1 heteromer is regulated in thrombo-inflammatory diseases. In COVID-19 patients, the frequency of TF-IFNAR1 heteromers on PBMCs was reduced in severe cases compared to mild cases (Figure 17D). This reduction correlated positively with plasma levels of CXCL10 (reflecting a type I interferon response, Figure 17E) and D-dimer (reflecting coagulation activation, Figure 17F). It was then scrutinized the TF-IFNAR1 interaction in a mouse model of myocardial infarction, likewise a thrombo-inflammatory disease. Pull-down of TF from myocardial tissues revealed readily detectable TF-IFNAR1 interaction in sham-operated mice, which was lost after myocardial ischemia-reperfusion injury (Figure 17G). Loss of the TF-IFNAR1 interaction was associated with increased heart IL-6 and plasma TAT levels (Figure 17H, I).

Next, fibroblast isolated from a patient with Aicardi-Goutières syndrome (AGS, an interferonopathy) associated with the TREX1^{V201D} mutation have been analyzed. Compared to wild-type (control) fibroblasts, the TF-IFNAR1 heteromer was markedly reduced on these fibroblasts (Figure 17J). This suggest that also genetic defects can disrupt the TF-IFNAR1 heteromer, inducing a sterile inflammatory response.

Collectively, these data support a model in which TF and IFNAR1 form an inactive heteromer in resting cells, but upon cellular stimulation the TF-IFNAR1 heteromer dissociates, inducing both TF-activity and IFNAR1-dependent signaling (Figure 17K).

## Claims

1. An interferon-a receptor (IFNAR1) immune modulator for use as a medicament for regulating the interaction of tissue factor (TF) and interferon-a receptor (IFNAR1) in a subject.

2. An IFNAR1 immune modulator for use as a medicament according to claim 1 for treating chronic inflammatory disease, autoimmune diseases and/or interferonopathy in a subject by regulating the interaction of TF and IFNAR1.

3. The modulator for use according to the preceding claim, wherein the subject has an interrupted interaction of TF and IFNAR1.

4. The modulator for use according to claim 2 or 3, wherein the administration of the modulator promotes an anti-inflammatory process.

5. The modulator for use according to any one of the claims 2-4, wherein the modulator binds to IFNAR1 so that the IFNAR1 signaling is suppressed.

6. The modulator for use according to any one of any one of the claims 2-5, wherein the administration of the inhibitor attenuates the IFNAR1/JAK/STAT signaling.

7. The modulator for use according to any one of the claims 2-6, wherein the modulator is an IFNAR1 inhibitor/antagonist and binds the D2-D4 domains of IFNAR1.

8. The modulator for use according to any one of the claims 2-7, wherein the modulator comprises at least one amino acid of tissue factor (110-242 aa of TF) according to SEQ ID NO:1, wherein the amino acid is selected from the group consisting of Q110, G120, T121, K149, I152, W158, S160, S161, S162, S163, G164, K166, K169, T170, N171, T172, E174, L176, D178, V179, D180, K181, C186, V192, S195, P206, V207, E208, C209 and E213 of the sequence according to SEQ ID NO 1, or a sequence having at least 70%, 75%, 80%, 85%, 90%, 95% sequence identity to SEQ ID NO: 1.

9. The modulator for use according to any one of the claims 2-8, wherein the subject has a medical condition associated with renal dysfunction.

10. The modulator for use according to any one of the claims 2-9, wherein the medical condition associated with renal dysfunction is a glomerular disease.

11. The modulator for use according to any one of the claims 2-10, wherein expression of TF is decreased in the glomerular disease.

12. The modulator for use according to any one of the claims 2-11, wherein the glomerular disease is associated with a reduced glomerular filtration rate (GFR), nephrotic syndrome and/or hypertension.

13. An IFNAR1 immune modulator for use as a medicament according to claim 1 for treating a tumor disease in a subject by regulating the interaction of TF and IFNAR1.

14. The modulator for use according to the preceding claim, wherein the administration of the modulator promotes the immune response to the tumoric antigen.

15. The modulator for use according to claim 13 or 14, wherein the modulator interrupts the interaction ofTF and IFNAR1.

16. The modulator for use according to any one of the claims 13-15, wherein the modulator is an IFNAR1 agonist which interrupts the binding of TF to D2-D4 domains of IFNAR1, wherein the modulator preferably comprises at least one amino acid of tissue factor (110-242 aa of TF) according to SEQ ID NO:1, wherein the amino acid is selected from the group consisting of Q110, G120, T121, K149, I152, W158, S160, S161, S162, S163, G164, K166, K169, T170, N171, T172, E174, L176, D178, V179, D180, K181, C186, V192, S195, P206, V207, E208, C209 and E213 of the sequence according to SEQ ID NO 1, or a sequence having at least 70%, 75%, 80%, 85%, 90%, 95% sequence identity to SEQ ID NO: 1.

17. The modulator for use according to any one of the preceding claims, wherein the modulator binds to IFNAR1 and maintains the immune homeostasis.

18. The modulator for use according to any one of the preceding claims, in combination with a pharmaceutically acceptable carrier.
